# EUROPEAN PATENT APPLICATION

(11) **EP 4 649 948 A1**
(43) Date of publication of application: **19.11.2025**
(21) Application number: 24741508.6
(22) Date of filing: 09.01.2024
(51) Int. Cl.: A61K 31/7088, A61P 9/00, A61P 9/04, A61P 43/00, C12N 5/071, C12N 15/12, C12Q 1/02, C12Q 1/6844, C12Q 1/6851, C12Q 1/686

(54) **CARDIAC DILATATION FUNCTION IMPROVING AGENT, AND METHOD FOR SCREENING FOR CARDIAC DILATATION FUNCTION IMPROVING AGENT**

(30) Priority: 10.01.2023 JP 2023001890
(71) Applicant: Keio University, Tokyo, 108-8345 (JP)
(72) Inventor: IEDA, Masaki, Tokyo 160-8582 (JP); YAMADA, Yu, Tsukuba-shi, Ibaraki 305-8577 (JP); SADAHIRO, Taketaro, Tsukuba-shi, Ibaraki 305-8577 (JP)
(74) Representative: Müller-Boré & Partner Patentanwälte PartG mbB
(86) International application number: PCT/JP2024/000151
(87) International publication number: WO 2024/150735

(57) **Abstract**

The cardiac diastolic function-improving agent according to an embodiment of the present invention comprises a polynucleotide encoding a reprogramming factor polypeptide Gata4.

## Description

### Technical Field

The present invention relates to a cardiac diastolic function-improving agent and a screening method for a cardiac diastolic function-improving agent.

### Background Art

Heart failure is classified into two types based on ejection fraction (EF): heart failure with reduced ejection fraction (HFrEF) and heart failure with preserved ejection fraction (HFpEF). Decreased cardiac diastolic function is known to be a common pathological condition in heart failure. For HFrEF, effective pharmacological and non-pharmacological treatments have been found. Although HFpEF is thought to be a complex pathological condition that involves fibrosis, cardiac hypertrophy, and inflammation, the detailed pathological condition remains to be elucidated, and the problem is that no effective treatments are available.

For example, PTL 1 discloses direct cardiac reprogramming to generate cardiomyocytes from fibroblasts in vivo. NPL 1 discloses suppressing fibrosis by introducing the GATA4 gene into fibroblasts after myocardial infarction.

### Citation List

### Patent Literature

PTL 1: WO2011/139688

### Non-patent Literature

NPL 1: J Thorac Cardiovasc Surg. 2017 Nov; 154(5): 1601-1610

### Summary of Invention

### Technical Problem

PTL 1 discloses a technique for transducing fibroblasts isolated from the hearts of α-myosin heavy chain-green fluorescent protein (αMHC-GFP) mice or Isl1-yellow fluorescent protein (Isl1-YFP) mice with a reprogramming gene containing Gata4, Mef2c, and Tbx5 to induce the fibroblasts to become cardiomyocytes; however, PTL 1 does not teach the effect of Gata4 on cardiac diastolic function in individuals. Direct cardiac reprogramming requires the introduction of three or more direct cardiac reprogramming genes into fibroblasts. Although the development of technology for simultaneously introducing genes into fibroblasts or the development of a vector capable of carrying multiple genes is necessary, it has not yet been realized.

In the technique disclosed in NPL 1, although fibrotic conditions in the heart were confirmed after the death of myocardial infarction model rats, an improvement in reduced cardiac diastolic function in living individuals has not yet been achieved. Therefore, further research and development is needed to improve reduced cardiac diastolic function.

An object of one aspect of the present invention is to provide a technique for improving cardiac diastolic function.

### Solution to Problem

A cardiac diastolic function-improving agent according to one aspect of the present invention comprises a polynucleotide encoding a reprogramming factor polypeptide Gata4.

In the cardiac diastolic function-improving agent according to one aspect of the present invention, the reprogramming factor polypeptide Gata4 comprises an amino acid sequence having at least 90% identity to the amino acid sequence represented by SEQ ID NO: 1 or SEQ ID NO: 3.

In the cardiac diastolic function-improving agent according to one aspect of the present invention, the polynucleotide comprises a nucleotide sequence having at least 90% identity to the nucleotide sequence represented by SEQ ID NO: 2 or SEQ ID NO: 4.

The cardiac diastolic function-improving agent according to one aspect of the present invention is an agent for improving heart failure.

In the cardiac diastolic function-improving agent according to one aspect of the present invention, a factor that upregulates the expression of Gata4 gene is present.

The cardiac diastolic function-improving agent according to one aspect of the present invention comprises a factor that upregulates the expression of Gata4 gene.

The cardiac diastolic function-improving agent according to one aspect of the present invention comprises a factor that increases the expression level of Gata4 gene in cardiac fibroblasts.

A screening method for the cardiac diastolic function-improving agent according to one aspect of the present invention comprises contacting a test substance with fibroblasts, and evaluating the expression of Gata4 gene in the fibroblasts.

### Advantageous Effects of Invention

According to one aspect of the present invention, a technique for improving cardiac diastolic function can be provided.

### Brief Description of Drawings

Fig. 1 shows a Tcf21 iCre mouse.
Fig. 2 shows a CAG-CAT-MGTH2A mouse.
Fig. 3 shows a tdTomato mouse.
Fig. 4 shows an immunostained cardiac section of a direct cardiac reprogramming mouse.
Fig. 5 shows quantitative PCR results from Gata4. The points indicate individual values of the samples.
Fig. 6 shows quantitative PCR results from Mef2c.
Fig. 7 shows quantitative PCR results from Tbx5.
Fig. 8 shows quantitative PCR results from Hand2.
Fig. 9 shows experimental protocols for HFpEF mouse model.
Fig. 10 shows systolic blood pressure measurement results from a normal chow group, an HFpEF group, and a reprogramming group.
Fig. 11 shows body weight measurement results from the normal chow group, the HFpEF group, and the reprogramming group.
Fig. 12 shows echocardiographic (M mode) results from the normal chow group, the HFpEF group, and the reprogramming group.
Fig. 13 shows echocardiographic (Doppler mode) results from the normal chow group, the HFpEF group, and the reprogramming group.
Fig. 14 shows temporal changes in EF.
Fig. 15 shows temporal changes in E/A.
Fig. 16 shows temporal changes in E/E'.
Fig. 17 shows the results (left ventricular pressure waveforms) of catheterization on mice.
Fig. 18 shows EDP measurement results.
Fig. 19 shows the results (PV loop waveforms) of catheterization on mice.
Fig. 20 shows EDPVR measurement results.
Fig. 21 shows evaluation using a mouse treadmill.
Fig. 22 shows the running distance of each group.
Fig. 23 shows immunostaining results from mouse cardiac sections. The images in the upper-right frames are enlarged images of the central portions in squares of the respective images. The scale bar is 50 µm.
Fig. 24 shows the cardiomyocyte induction efficiency of each group.
Fig. 25 shows cardiac macroimages and immunostained images of cardiac sections.
Fig. 26 shows measurement results of the ratio of cardiac weight corrected for femur length.
Fig. 27 shows cardiomyocyte area measurement results.
Fig. 28 shows cardiac section Sirius red staining results.
Fig. 29 shows the fibrosis area of each group.
Fig. 30 shows scRNA-seq results from non-cardiomyocytes in the heart.
Fig. 31 shows analysis results from interaction between non-cardiomyocytes in the heart.
Fig. 32 shows a comprehensive score of pathogenic genes whose expression is increased due to HFpEF on the vertical axis.
Fig. 33 shows a heatmap for expression of representative gene clusters that are activated in response to cardiac impairment.
Fig. 34 shows an ATAC-seq experimental workflow.
Fig. 35 shows an ATAC-seq experimental workflow.
Fig. 36 shows a heatmap for signal intensity in the region where the peak wave height was significantly different in ATAC-seq.
Fig. 37 shows Gata4 ChIP-seq data.
Fig. 38 shows a heatmap for Gata4 ChIP-seq data.
Fig. 39 shows GO analysis results.
Fig. 40 shows an overview of a genetically modified mouse.
Fig. 41 shows quantitative PCR measurement results from Gata4, Mef2c, Tbx5, and Hand2.
Fig. 42 shows quantitative PCR measurement results from Col1a2, Fn1, and Postn.
Fig. 43 shows FACS results.
Fig. 44 shows an overview of genetically modified mice.
Fig. 45 shows experimental protocols.
Fig. 46 shows temporal changes in body weight, systolic blood pressure (SBP), and diastolic blood pressure (DBP).
Fig. 47 shows echocardiographic results.
Fig. 48 shows the treadmill running distance.
Fig. 49 shows cardiac catheterization results.
Fig. 50 shows cardiac section Sirius red staining results.
Fig. 51 shows the fibrosis area of each group.
Fig. 52 shows quantitative PCR results from Col1a1, Col3a1, Nppd, and Tgfb1.
Fig. 53 summarizes the results of the Examples.

### Description of Embodiments

### Definition of Terms etc.

In the present specification, the term "polynucleotide" can also be referred to as "nucleic acid" or "nucleic acid molecule," and is intended to mean a polymer of nucleotides. The term "base sequence" can also be referred to as "nucleic acid sequence" or "nucleotide sequence." Unless otherwise specified, polynucleotides can exist in the form of RNA or DNA. The form of RNA includes, for example, mRNA. The form of DNA includes, for example, cDNA or genomic DNA. The DNA may be double-stranded or single-stranded.

In the present specification, the term "protein" can also be referred to as "polypeptide."

Proteins disclosed in the present specification may be, but are not limited to, polypeptides in which amino acids are linked by peptide bonds, and may also be those having structures other than polypeptides. Examples of the structures other than polypeptides as used herein include, but are not limited to, sugar chains and isoprenoid groups.

In the present specification, the phrase "A and/or B" represents a concept that includes both "A and B" and "A or B," and can be rephrased as "at least one of A or B."

In the present specification, "improving cardiac diastolic function" includes reducing or alleviating a decline in cardiac diastolic function or the risk of the decline, delaying the progression of a decline in cardiac diastolic function, healing a decline in cardiac diastolic function, enhancing cardiac diastolic function, and the like.

In the present specification, the terms "comprise" and "contain" also include the concepts of consisting essentially of and consisting of.

The upper or lower limit of numerical ranges stated in the present specification may be replaced with values shown in the Examples or values that can be unambiguously derived from the Examples. Further, in the present specification, numerical values connected by "to" indicate a range in which the first value before "to" represents the lower limit and the second value after "to" represents the upper limit.

### Cardiac Diastolic Function-improving Agent

A cardiac diastolic function-improving agent according to one aspect of the present invention comprises a polynucleotide encoding a reprogramming factor polypeptide Gata4 (sometimes referred to below as "the Gata4 polynucleotide").

The myocardial infarction rats used in NPL 1 are a rat model of ischemic heart disease that is different from HFpEF, and the cardiac fibrosis pattern is different from that revealed in this study. It was thus impossible before the filing date of this application to infer, based on the disclosure of NPL 1, whether Gata4 would have a therapeutic effect on cardiac fibrosis in an animal model with a completely different pattern in the first place.

Although it has been suggested that cardiac diastolic dysfunction has a variety of causes, including not only cardiac fibrosis but also cardiomyocyte hypertrophy, endothelial dysfunction, and inflammation, it remains unclear whether addressing these causes would lead to an improvement in diastolic function.

The present inventors have found that fibrotic cell intervention produces an effect of improving cardiac diastolic function, which cannot be expected by inhibiting fibrosis in a conventional manner.

### Gata4

Gata4 polypeptides are members of the GATA family of zinc-finger transcription factors, which recognize and bind to a GATA motif present in the promoter regions of many genes (e.g., recognize and bind to the consensus sequence 5'-AGATAG-3'). Gata4 is described, for example, in Huang et al., Gene, 1995, 155(2): 219-23. The amino acid sequences related to Gata4 polypeptides from various species and the nucleotide sequences encoding Gata4 polypeptides are known in the related fields. Examples of amino acid sequences related to Gata4 polypeptides and the accession numbers of nucleotide sequences encoding Gata4 polypeptides are shown below.

### Examples of Amino Acid Sequences Related to Gata4 Polypeptides

- NP_002043 (*Homo sapiens;* SEQ ID NO: 1)
- NP_0321188 (*Mus musculus;* SEQ ID NO: 3)
- NP_653331 (*Rattus norvegicus*)
- ABI63575 (*Danio rerio*)
- AAH71101 (*Xenopus laevis*)

### Examples of Nucleotide Sequences Encoding Gata4 polypeptides

- CDS sequence in NM_002052 (*Homo sapiens*) (SEQ ID NO: 2)
- CDS sequence in NM_008092 (*Mus musculus*) (SEQ ID NO: 4)
- CDS sequence in NM_144730 (*Rattus norvegicus*)
- CDS sequence in DQ886664 (*Danio rerio*)
- CDS sequence in BC071107 (*Xenopus laevis*)

In some embodiments, the Gata4 polypeptide comprises an amino acid sequence having at least 90%, at least 95%, at least 96%, at least 97%, at least 98%, at least 99%, or 100% identity to the amino acid sequence represented by SEQ ID NO: 1. In some embodiments, the Gata4 polypeptide comprises an amino acid sequence having at least 90%, at least 95%, at least 96%, at least 97%, at least 98%, at least 99%, or 100% identity to the amino acid sequence represented by SEQ ID NO: 3. The Gata4 polypeptides are biologically active, and recognize and bind to, for example, a GATA motif present in a promoter (e.g., recognize and bind to the consensus sequence 5'-AGATAG-3'), activating the transcription of a gene operably linked to the promoter containing the GATA motif.

In some embodiments, the polynucleotide encoding Gata4 comprises a nucleotide sequence having at least 90%, at least 95%, at least 96%, at least 97%, at least 98%, at least 99%, or 100% identity to the nucleotide sequence represented by SEQ ID NO: 2. In some embodiments, the polynucleotide encoding Gata4 comprises a nucleotide sequence having at least 90%, at least 95%, at least 96%, at least 97%, at least 98%, at least 99%, or 100% identity to the nucleotide sequence represented by SEQ ID NO: 4.

In some embodiments, polypeptides that are functionally equivalent to Gata4 polypeptides (or nucleotide sequences encoding such functional equivalents) are used. For example, in some embodiments, a Gata5 polypeptide (or a nucleotide sequence encoding a Gata5 polypeptide) is used. In another embodiment, a Gata6 polypeptide (or a nucleotide sequence encoding a Gata6 polypeptide) is used.

The amino acid sequences of Gata5 polypeptides and the nucleotide sequences encoding Gata5 polypeptides are known in the related fields. Examples of amino acid sequences related to Gata5 polypeptides and the accession numbers of nucleotide sequences encoding Gata5 polypeptides are shown below.

### Examples of Amino Acid Sequences Related to Gata5 Polypeptides

- NP_536721 (*Homo sapiens*)
- NP_032119 (*Mus musculus*)
- NP_001019487 (*Rattus norvegicus*)

### Examples of Nucleotide Sequences Encoding Gata5 Polypeptide

- CDS sequence in NM_080473 (*Homo sapiens*)
- CDS sequence in NM_008093 (*Mus musculus*)
- CDS sequence in NM_001024316 (*Rattus norvegicus*)

The amino acid sequences of Gata6 polypeptides and the nucleotide sequences encoding Gata6 polypeptides are known in the related fields. Examples of amino acid sequences related to Gata6 polypeptides and the accession numbers of nucleotide sequences encoding Gata6 polypeptides are shown below.

### Examples of Amino Acid Sequences Related to Gata6 polypeptides

- NP_005248 (*Homo sapiens*)
- NP_034388 (*Mus musculus*)
- NP_062058 (*Rattus norvegicus*)

### Examples of Nucleotide Sequences Encoding Gata6 Polypeptides

- CDS sequence in NM_005257 (*Homo sapiens*)
- CDS sequence in NM_010258 (*Mus musculus*)
- CDS sequence in NM_019185 (*Rattus norvegicus*)

In some embodiments, a suitable functional equivalent of the Gata4 polypeptide is a polypeptide having at least 90%, at least 95%, at least 96%, at least 97%, at least 98%, at least 99%, or 100% identity to the amino acid sequence of the Gata5 polypeptide or Gata6 polypeptide.

In some embodiments, the nucleotide sequence encoding a functional equivalent of the Gata4 polypeptide comprises a nucleotide sequence having at least 90%, at least 95%, at least 96%, at least 97%, at least 98%, at least 99%, or 100% identity to the nucleotide sequence encoding the Gata5 polypeptide or Gata6 polypeptide.

The method for obtaining (isolating) the Gata4 polynucleotide is not particularly limited. For example, the Gata4 polynucleotide may be synthesized according to a nucleic acid synthesis method, such as the phosphoramidite method.

Examples of a method for obtaining the Gata4 polynucleotide include a method that uses nucleic acid amplification, such as PCR. For example, primers are prepared from the 5' and 3' end sequences (or their complementary sequences) of cDNA of the polynucleotide, and PCR or the like is performed using these primers with genomic DNA, cDNA or the like, as the template to amplify the DNA region sandwiched between these primers. This makes it possible to obtain a large amount of DNA fragments containing the polynucleotide according to the present invention.

One aspect of the present invention also includes a cardiac diastolic function-improving agent comprising the Gata4 polynucleotide (e.g., DNA). In one preferred embodiment of the cardiac diastolic function-improving agent, the Gata4 polynucleotide (e.g., DNA) is inserted into a vector. The vector may be, for example, an autonomously replicating vector (e.g., a plasmid) or may be a vector that integrates into the host cell genome upon introduction into a host cell and replicates along with the chromosome into which it has been integrated.

The vector is preferably an expression vector. In the expression vector, elements necessary for transcription (e.g., a promoter, enhancer, ribosome-binding site, splice signal, and terminator) are functionally linked to the Gata4 polynucleotide.

Examples of vectors include viral vectors, such as retroviral vectors, adenoviral vectors, adeno-associated viral vectors (AAV vectors), Sendai viral vectors, and lentiviral vectors; and non-viral vectors, such as plasmid vectors, bacterial vectors, phage vectors, phagemid vectors, and cosmid vectors.

The vector can be constructed, for example, by using known genetic engineering techniques. In one example, the vector expresses the Gata4 polynucleotide specifically in myocardial fibroblasts.

One aspect of the present invention also includes a cardiac diastolic function-improving agent comprising a fibroblast with an introduced Gata4 polynucleotide (e.g., mRNA) or with an introduced vector into which the Gata4 polynucleotide has been inserted. The administration of Gata4-expressing cells to a subject can improve cardiac diastolic function.

Examples of fibroblasts include cardiac fibroblasts. Fibroblasts can be obtained from living individuals, for example, from tissues collected from living individuals. Fibroblasts can be isolated from tissues using known techniques. For example, the method described in Ieda et al., Dev Cell., 2009, 16(2), 233-244 or the method described in the Examples can be used. The collected fibroblasts may be cells collected from the same individual of the same species as the subject to be administered (autologous cells), or may be cells collected from a different individual of the same species as the subject to be administered (allogeneic cells).

The introduction, into fibroblasts, of the Gata4 polynucleotide or a vector into which the Gata4 polynucleotide has been inserted may be performed by, for example, electroporation methods, calcium phosphate methods, lipofection methods, microinjection methods, introduction methods using liposomes, introduction methods using gene guns, and introduction methods using cationic polymers (e.g., DEAE dextran, polyethyleneimine, and polyethylene glycol).

One aspect of the present invention also includes a cardiac diastolic function-improving agent comprising a factor that upregulates the expression of Gata4 gene. Examples of the factor that upregulates the expression of Gata4 gene include Gata4-binding factors, such as RbAp46 (retinoblastoma protein-associated protein 46) and RbAp48 (retinoblastoma protein-associated protein 48). One aspect of the present invention also includes a cardiac diastolic function-improving agent comprising a factor that increases the expression level of Gata4 gene in cardiac fibroblasts. Examples of the factor that increases the expression level of Gata4 gene include Gata4-binding factors, such as RbAp46 (retinoblastoma protein-associated protein 46) and RbAp48 (retinoblastoma protein-associated protein 48).

### Other Components

The cardiac diastolic function-improving agent according to one aspect of the present invention may further comprise other components, in addition to the Gata4 polynucleotide, the factor that upregulates the expression of Gata4 gene, or the factor that increases the expression level of Gata4 gene in cardiac fibroblasts. Examples of such other components include, but are not particularly limited to, pharmaceutically acceptable carriers, lubricants, preservatives, stabilizers, wetting agents, emulsifiers, salts for adjusting osmotic pressure, buffers, stabilizers, preservatives, excipients, antioxidants, viscosity modifiers, colorants, flavors, and sweeteners. When the cardiac diastolic function-improving agent is formed as an aqueous solution, it is possible to use pure water (sterile water), physiological saline, phosphate buffered saline, or the like as the carrier. When the cardiac diastolic function-improving agent is formed as another suitable solution, it is possible to use an organic ester capable of being introduced in vivo, such as glycol, glycerol, or olive oil, as the carrier. The cardiac diastolic function-improving agent comprising the Gata4 polynucleotide, the factor that upregulates the expression of Gata4 gene, or the factor that increases the expression level of Gata4 gene in cardiac fibroblasts, and comprising one or more other components is also referred to as "a composition for improving cardiac diastolic function."

The cardiac diastolic function-improving agent according to one aspect of the present invention may be contained in a container, pack, dispenser, or the like, together with instructions for use.

### Subject and Administration of Cardiac Diastolic Function-improving Agent

Examples of subjects to which the cardiac diastolic function-improving agent according to one aspect of the present invention is administered include humans and non-human animals. More specific examples include vertebrates, such as birds and mammals. Mammals include laboratory animals, such as mice, rats, rabbits, guinea pigs, and non-human primates; companion animals (pets), such as dogs and cats; farm animals, such as pigs, cows, goats, sheep, and horses; and humans.

The cardiac diastolic function-improving agent according to one aspect of the present invention is preferably used to improve heart failure. The cardiac diastolic function-improving agent can be an agent for improving heart failure. Examples of heart failure include heart failure with reduced ejection fraction (HFrEF) and heart failure with preserved ejection fraction (HFpEF). The cardiac diastolic function-improving agent can be used to improve both HFrEF and HFpEF.

The administration route or administration method is not particularly limited. The agent may be administered directly to the subject's heart or to the vicinity thereof, or may be administered indirectly. Examples of the administration route include oral, intravenous, intramuscular, subcutaneous, intraventricular, intraperitoneal, and transdermal routes. The administration can also be performed, for example, by local administration or according to methods using a gene gun.

The dose and frequency of administration can be appropriately selected according to the severity of symptoms, age, gender, body weight, administration form, or the like.

### Method for Improving Cardiac Diastolic Function

One aspect of the present invention also includes a method for improving cardiac diastolic function, comprising administering the Gata4 polynucleotide to a subject in need of improvement of cardiac diastolic function.

One aspect of the present invention also includes a method for improving cardiac diastolic function, comprising administering a factor that upregulates the expression of Gata4 gene or a factor that increases the expression level of Gata4 gene in cardiac fibroblasts to a subject in need of improvement of cardiac diastolic function.

The above methods may further comprise introducing the Gata4 polynucleotide into fibroblasts before the administration step. The fibroblasts may be those isolated from the same individual as the subject to which the Gata4 polynucleotide is administered or from a different individual of the same species. Alternatively, the fibroblasts may be cultured cells derived from the same individual as the subject to which the Gata4 polynucleotide is administered or from a different individual of the same species. The subject to which the Gata4 polynucleotide is administered is as described above in terms of the subjects to which the cardiac diastolic function-improving agent is administered. The Gata4 polynucleotide may also be introduced in the form of a vector. Therefore, one aspect of the present invention also includes a method for producing fibroblasts for introduction into a subject, the method comprising introducing the Gata4 polynucleotide into fibroblasts.

The above method may further comprise introducing into the fibroblasts a factor that upregulates the expression of Gata4 gene or a factor that increases the expression level of Gata4 gene in cardiac fibroblasts. The fibroblasts may be those isolated from the same individual as the subject to which the Gata4 polynucleotide is administered or from a different individual of the same species. Alternatively, the fibroblasts may be cultured cells derived from the same individual as the subject to which the Gata4 polynucleotide is administered or from a different individual of the same species. The factor that upregulates the expression of Gata4 gene or a factor that increases the expression level of Gata4 gene in cardiac fibroblasts may also be introduced in the form of a vector. Therefore, one aspect of the present invention also includes a method for producing fibroblasts for introduction into a subject, the method comprising introducing into fibroblasts a factor that upregulates the expression of Gata4 gene or a factor that increases the expression level of Gata4 gene in cardiac fibroblasts.

### Screening Method for Cardiac Diastolic Function-improving Agent

A screening method for the cardiac diastolic function-improving agent according to one aspect of the present invention comprises a contacting step and an evaluation step.

### Contacting Step

The contacting step includes contacting a test substance with fibroblasts. Examples of fibroblasts include cardiac fibroblasts. The cells may be obtained from a living individual, for example, from tissues collected from a living individual. The living individual may be an individual with normal cardiac diastolic function or an individual with reduced cardiac diastolic function. The fibroblasts can be isolated from tissues using known techniques.

The screening method may further comprise the step of introducing the Gata4 polynucleotide into fibroblasts before the contacting step. The Gata4 polynucleotide may be introduced in the form of a vector. In this case, contacting the test substance with fibroblasts includes contacting the test substance with fibroblasts into which the Gata4 polynucleotide has been introduced or with a vector containing such fibroblasts. Specific methods for introduction include those described in the "Cardiac Diastolic Function-improving Agent" section.

### Evaluation Step

In the evaluation step, the expression of Gata4 gene in fibroblasts is evaluated after the contacting step. Specifically, the expression level of Gata4 gene or the amount of the polypeptide Gata in the fibroblasts after the contacting step is measured. Examples of the measurement method include RT-PCR, hybridization analysis, and molecular biology methods.

The screening method may also comprise a comparison step of comparing the expression level (e.g., expression amount) of Gata4 gene in the fibroblasts after the contacting step with the expression level of Gata4 gene in the fibroblasts before the contacting step. If the expression of Gata4 gene in the fibroblasts after the contacting step is upregulated compared to the expression of Gata4 gene in the fibroblasts before the contacting step, the test substance may serve as a candidate for the cardiac diastolic function-improving agent. The test substance can be selected as a candidate for the cardiac diastolic function-improving agent.

Alternatively, the screening method may further comprise a comparison step of comparing the expression level (e.g., expression amount) of the polypeptide Gata in the fibroblasts after the contacting step with the expression level (e.g., expression amount) of the polypeptide Gata in the fibroblasts before the contacting step. If the expression of the polypeptide Gata in the fibroblasts after the contacting step is upregulated compared to the expression of the polypeptide Gata in the fibroblasts before the contacting step, the test substance can serve as and can be selected as a candidate for the cardiac diastolic function-improving agent.

### Summary

A cardiac diastolic function-improving agent according to aspect 1 of the present invention comprises a polynucleotide encoding a reprogramming factor polypeptide Gata4.

A cardiac diastolic function-improving agent according to aspect 2 of the present invention is the cardiac diastolic function-improving agent according to aspect 1 of the present invention, wherein the polynucleotide encoding a reprogramming factor polypeptide Gata4 comprises a polynucleotide encoding a polypeptide comprising an amino acid sequence having at least 90% identity to the amino acid sequence represented by SEQ ID NO: 1.

A cardiac diastolic function-improving agent according to aspect 3 of the present invention is the cardiac diastolic function-improving agent according to aspect 1 of the present invention, wherein the polynucleotide encoding a reprogramming factor polypeptide Gata4 comprises a polynucleotide encoding a polypeptide that comprises an amino acid sequence having at least 90% identity to the amino acid sequence represented by SEQ ID NO: 1, and that recognizes and binds to a GATA motif present in a promoter.

A cardiac diastolic function-improving agent according to aspect 4 of the present invention is the cardiac diastolic function-improving agent according to aspect 1 of the present invention, wherein the polynucleotide encoding a reprogramming factor polypeptide Gata4 comprises a polypeptide comprising the amino acid sequence represented by SEQ ID NO: 1.

A cardiac diastolic function-improving agent according to aspect 5 of the present invention is the cardiac diastolic function-improving agent according to aspect 1 of the present invention, wherein the polynucleotide encoding a reprogramming factor polypeptide Gata4 comprises a polynucleotide encoding a polypeptide comprising an amino acid sequence having at least 90% identity to the amino acid sequence represented by SEQ ID NO: 3.

A cardiac diastolic function-improving agent according to aspect 6 of the present invention is the cardiac diastolic function-improving agent according to aspect 1 of the present invention, wherein the polynucleotide encoding a reprogramming factor polypeptide Gata4 comprises a polynucleotide encoding a polypeptide that comprises an amino acid sequence having at least 90% identity to the amino acid sequence represented by SEQ ID NO: 3, and that recognizes and binds to a GATA motif present in a promoter.

A cardiac diastolic function-improving agent according to aspect 7 of the present invention is the cardiac diastolic function-improving agent according to aspect 1 of the present invention, wherein the polynucleotide encoding a reprogramming factor polypeptide Gata4 comprises a polypeptide comprising the amino acid sequence represented by SEQ ID NO: 3.

A cardiac diastolic function-improving agent according to aspect 8 of the present invention is the cardiac diastolic function-improving agent according to aspect 1 of the present invention, wherein the polynucleotide encoding a reprogramming factor polypeptide Gata4 comprises a polynucleotide comprising a nucleotide sequence having at least 90% identity to the nucleotide sequence represented by SEQ ID NO: 2.

A cardiac diastolic function-improving agent according to aspect 9 of the present invention is the cardiac diastolic function-improving agent according to aspect 1 of the present invention, wherein the polynucleotide encoding a reprogramming factor polypeptide Gata4 comprises a polynucleotide that comprises a nucleotide sequence having at least 90% identity to the nucleotide sequence represented by SEQ ID NO: 2, and that encodes a polypeptide that recognizes and binds to a GATA motif present in a promoter.

A cardiac diastolic function-improving agent according to aspect 10 of the present invention is the cardiac diastolic function-improving agent according to aspect 1 of the present invention, wherein the polynucleotide encoding a reprogramming factor polypeptide Gata4 comprises the nucleotide sequence represented by SEQ ID NO: 2.

A cardiac diastolic function-improving agent according to aspect 11 of the present invention is the cardiac diastolic function-improving agent according to aspect 1 of the present invention, wherein the polynucleotide encoding a reprogramming factor polypeptide Gata4 comprises a polynucleotide comprising a nucleotide sequence having at least 90% identity to the nucleotide sequence represented by SEQ ID NO: 4.

A cardiac diastolic function-improving agent according to aspect 12 of the present invention is the cardiac diastolic function-improving agent according to aspect 1 of the present invention, wherein the polynucleotide encoding a reprogramming factor polypeptide Gata4 comprises a polynucleotide that comprises a nucleotide sequence having at least 90% identity to the nucleotide sequence represented by SEQ ID NO: 4, and that encodes a polypeptide that recognizes and binds to a GATA motif present in a promoter.

A cardiac diastolic function-improving agent according to aspect 13 of the present invention is the cardiac diastolic function-improving agent according to aspect 1 of the present invention, wherein the polynucleotide encoding a reprogramming factor polypeptide Gata4 comprises the nucleotide sequence represented by SEQ ID NO: 4.

A cardiac diastolic function-improving agent according to aspect 14 of the present invention comprises a factor that upregulates the expression of Gata4 gene.

A cardiac diastolic function-improving agent according to aspect 15 of the present invention comprises a factor that increases the expression level of Gata4 gene in cardiac fibroblasts.

A cardiac diastolic function-improving agent according to aspect 16 of the present invention is the cardiac diastolic function-improving agent according to any one of aspects 1 to 3 of the present invention, which is an agent for improving heart failure.

A cardiac diastolic function-improving agent according to aspect 17 of the present invention is the cardiac diastolic function-improving agent according to aspect 4 of the present invention, wherein the heart failure is heart failure with preserved ejection fraction (HFpEF).

A screening method for the cardiac diastolic function-improving agent according to aspect 18 of the present invention comprises a contacting step of contacting a test substance with fibroblasts, and an evaluation step of evaluating the expression of Gata4 gene in the fibroblasts.

Embodiments of the present invention are described in more detail with reference to the Examples below. Of course, the present invention is not limited to the Examples below, and various possible aspects are included in detail. Furthermore, the present invention is not limited to the embodiments described above, and various modifications are possible within the scope of the claims. Embodiments obtained by appropriately combining the technical means disclosed herein are also included in the technical scope of the present invention. In addition, all of the documents cited in the present specification are incorporated herein by reference.

### Examples

All of the experiments described below were conducted with the approval of the Ethics Committees for Animal Experiments of the Tsukuba University.

### Material and Methods

### Mice

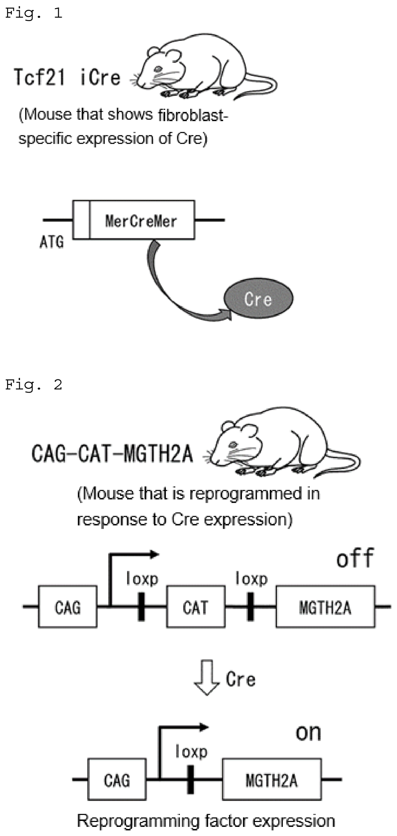

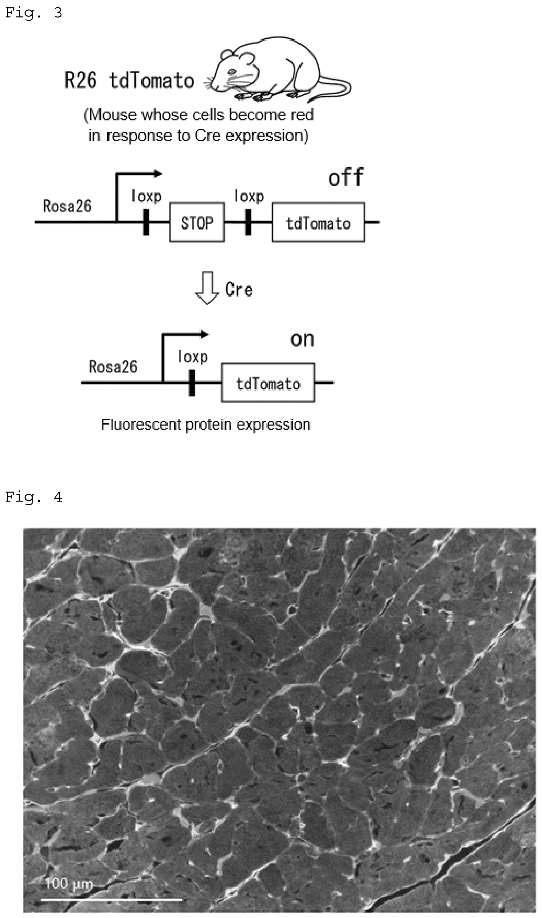

Tcf21 iCre/tdTomato mice were obtained by crossing Tcf21 iCre mice (Fig. 1) with R26 tdTomato reporter mice (Fig. 3). Cre-responsive MGTH2A (a gene sequence of Mef2c, Gata4, Tbx5, and Hand2 linked with self-cleaving peptide 2A) transgenic mice were constructed by modifying a plasmid with the CAG-CAT-Z structure (Fig. 2, CAG-CAT-MGTH2A mice). This plasmid contains a CMV (Cytomegalovirus) enhancer and a CAG (Chicken beta-actin) promoter and is linked to the CAT (Chloramphenicol acetyltransferase) gene surrounded by loxp sequences. The full-length mouse MGTH2A gene sequence was generated by ligating T2A and Hand2 to the Mef2c-Gata4-Tbx5 gene sequence, and inserted into the Z site of the plasmid with the CAG-CAT-Z structure. The constructed plasmid was purified, linearized, and then introduced by microinjection into the pronuclei of blastocyst-stage fertilized eggs of FVBN mice. The transgenic mice were verified for the presence of CAT using DNA PCR. The CAG-CAT-MGTH2A mice were crossed with the Tcf21 iCre/tdTomato mice to produce triple transgenic mice.

Single-factor mice for each of Gata4, Mef2c, Tbx5, and Hand2 (sometimes abbreviated below using their first letters: "G," "M," "T," and "H," respectively) were constructed using plasmids with the CAG-LSL(loxp-Stop-loxp)-Z structure in which a CAG promoter and SV40pA surrounded by loxp sequences were present within the Rosa26 locus homologous sequence. The plasmids were produced by inserting the G, M, T, or H gene sequence individually into the Z site of CAG-LSL-Z. The constructed plasmids were purified, linearized, and then introduced into ICR mouse fertilized eggs. The target gene sequence was inserted into the Rosa26 locus through homologous recombination. Introduction of the inserted gene into Rosa26 and gene insertion into other gene loci were confirmed using DNA PCR, and individuals with gene insertion only at the target Rosa26 locus were used for the experiments. The CAG-LSL-Single factor (G, M, T, H) mice were crossed with the Tcf21 iCre mice to produce double transgenic mice.

No immune deficiencies or other health problems were observed in the transgenic mice, and all animals were naive to experimental assessment and drug treatment until the start of the experiment. All animals were group-housed in a dedicated specific pathogen-free (SPF) facility with 12 h/12 h light-dark cycles, had ad libitum access to food and water, and were checked daily. Regular health checks were conducted to maintain the SPF grade.

### HFpEF Model Mice

HFpEF (heart failure preserved ejection fraction) model mice were produced by continuous administration of L-NAME (Nω-nitro-L-arginine methyl ester, N5751, Sigma) and a high-fat diet (HFD32, CLEA Japan) in accordance with a previous report (Schiattarella GG et al., Nature, 2019). L-NAME was dissolved in tap water at a concentration of 1.0 g/L and the mice were allowed to drink water ad libitum from a water bottle. The L-NAME and high-fat diet were exchanged every 2 or 3 days.

### Induction of Cre by Tamoxifen Administration

Tamoxifen (T5648, Sigma) was administered by intraperitoneal injection to the mice at a dose of 2 mg/day for 5 consecutive days, starting from the fifth week following the start of the experiment. Tamoxifen was dissolved for use in peanut oil (90%; P2144, Sigma) and ethanol (10%) at a concentration of 50 mg/mL.

### Mouse Fibroblasts

Mouse embryonic fibroblasts (MEFs) were isolated as follows. Embryos isolated from pregnant mice at 12.5 days of gestation were washed with phosphate-buffered saline (PBS), and the head and visceral tissues were then carefully removed. The remaining embryo parts were each washed with fresh PBS, minced with scissors, transferred to a 0.25% trypsin/ethylenediaminetetraacetic acid solution (25200-072, Gibco), and incubated at 37°C for 15 minutes. After trypsinization, an equal volume of fetal bovine serum (FBS, SV30014.03, Thermo Scientific) was added, and the tissue was dissociated by pipetting several times. The lysate was transferred into a fresh tube, and the cells were collected by centrifugation. The cells were them resuspended in DMEM/10% FBS (Dulbecco's modified Eagle's medium DMEM containing 10% FBS, 044-29765, FUJIFILM) and cultured at 37°C in 5% CO₂. The genotype of MEFs was analyzed using DNA PCR with the head and visceral tissues.

### Echocardiography

Transthoracic echocardiographic analysis was performed using a Vevo 2100 system (VisualSonics). The mice were anesthetized with low-dose isoflurane (1.0 to 2.0%) for echocardiography. During echocardiographic imaging, the body temperature was controlled with a warming pad while electrocardiogram monitoring was performed using limb electrodes. For cardiac contractility, left ventricular ejection fraction (LVEF) was evaluated by measuring the left ventricular end-diastolic dimension (LVDd) and the left ventricular end-systolic dimension (LVDs) using M-mode echo at the papillary muscle level in the left ventricular short-axis view. In addition, mitral valve inflow velocity waveforms were obtained using Doppler echo from the apical four-chamber view, and the early diastolic motion velocity peak value (E') of the mitral annulus was measured using tissue Doppler imaging. LVEF was calculated according to the following equation 1 using the Teichholz equation. EF = (left ventricular end-diastolic volume - left ventricular end-systolic volume)/left ventricular end-diastolic volume × 100 (%)

The left ventricular end-diastolic volume and the left ventricular end-systolic volume were calculated according to the following equations 2 and 3, respectively. Left ventricular end-diastolic volume = [{7.0/(2.4 + LVDd)} × (LVDd)3] Left ventricular end-systolic volume = [{7.0/(2.4 + LVDs)} × (LVDs)3]

### Blood Pressure Measurement

Systolic and diastolic blood pressures were measured non-invasively in conscious mice using a mouse tail blood pressure measuring device (CODA, Kent Scientific). Mice were placed in individual holders on a temperature-controlled platform (37°C) and recordings were performed under steady-state conditions. Before testing, all mice were trained to acclimatize to short-term restraint. The blood pressure was measured at least 10 times, and the average value was used.

### Cardiac Catheterization

Cardiac catheterization was performed via a right internal carotid artery approach using a mouse conductance catheter (SPR-839, Millar) and a Pressure-Volume System (MPVS-400, Millar). The mice were anesthetized with isoflurane and placed on a warmer. Electrocardiogram monitoring was performed using limb electrodes. The right internal carotid artery was exposed via an incision, and a conductance catheter was inserted. The tip was inserted into the left ventricle, and the left ventricular pressure waveform in a steady state was recorded. Next, a small incision was made in the upper abdomen to identify the inferior vena cava. The pressure-volume loop (PV loop) waveform during inferior vena cava occlusion was recorded by applying pressure to the inferior vena cava with a cotton swab. Analysis was performed using LabChart (ADInstruments).

### Mouse Treadmill

The mouse treadmill was performed using a belt-type forced running machine (TMS-4N, MELQUEST). For 2 days before the start of the test, the mice were acclimatized to treadmill exercise (0°, 10 m/min, 10 min). The test was conducted such that the mice ran at a warm-up speed of 5 m/min for 4 minutes at a 20° uphill incline, the speed was then increased to 14 m/min for 2 minutes, the speed was then increased by 2 m/min every 2 minutes until the mice reached exhaustion, and the total running distance was measured. Exhaustion was defined as the inability to resume running within 10 seconds after contact with the rear electrical stimulation grid.

### Fluorescence-activated Cell Sorting

To detect cTnT expression using fluorescence-activated cell sorting (FACS), cells were fixed in 4% PFA for 15 minutes, permeabilized with saponin (47036-250G-F, Sigma Aldrich), stained with anti-cTnT (MS-295-P1, Thermo Scientific) antibody, and then incubated with a secondary antibody conjugated with Alexa Fluor 488 (A11001, Invitrogen). The cells were then analyzed using a FACS instrument (CytoFLEX S, Beckman Coulter) and FlowJo software (Tomy Digital Biology). Non-cardiomyocytes and Tomato-positive cells were collected using BD FACSAria^{™} IIIu (BD) and MoFlo XDP (Beckman Coulter).

### Histological Testing

After the mice were euthanized, the hearts were perfused from the apex with PBS and 0.4% paraformaldehyde (PFA) in this order. The hearts were then quickly harvested and fixed overnight in 4% PFA. For immunostaining of frozen sections, frozen blocks were prepared in liquid nitrogen by embedding them in OCT compound after replacement with a 20% sucrose solution. Using a microtome, the hearts were cut vertically into 7 µm sections to expose both ventricles. The frozen sections were stained with primary antibodies against α-actinin (A7811, Sigma), followed by secondary antibodies conjugated with Alexa488 and DAPI. Wheat germ agglutinin (WGA) staining was performed using antibodies conjugated with Alexa488. Confocal microscopy was performed using an LSM800 microscope (Carl Zeiss). The ratio of α-Actinin+/Tomato+ cells was measured by counting in ten randomly selected fields from five or more different sections of each mouse.

For preparation of paraffin sections, paraffin-embedded sections were prepared from the hearts after fixation, followed by staining with Sirius red or wheat germ agglutinin (WGA). For WGA staining, antibodies conjugated with Alexa488 (W11261, Thermo Scientific) were used. Measurement of the fibrosis area and the cross-sectional area of cardiomyocytes was performed using Image J (NIH). All measurements and calculations were performed under blinded conditions.

### DNA PCR and qRT-PCR

The genotype of transgenic mice was determined by the standard PCR using the primers shown in Table 1. Total RNA was extracted using standard protocols from fibroblasts transduced in vitro, cardiac cells from both ventricles, and isolated Tomato-positive cells. qRT-PCR was performed with the primers and TaqMan probes (Applied Biosystems) shown in Tables 2 and 3 using a StepOnePlus Real-Time PCR system. Table 2 shows the details of a TaqMan gene expression assay (Applied Biosystems, Thermo Fischer Scientific). Table 2 shows the details of a Universal Probe Library System (Roche). The expression of target mRNA was corrected by the expression of Gapdh.

**Table 1**

| Primer name | Sequence |
|---|---|
| *CA T-forward* | CAGTCAGTTGCTCAATGTACC (SEQ ID NO:5) |
| *CAT*-reverse | ACTGGTGAAACTCACCCA (SEQ ID NO:6) |
| *Cre*-forward | GTTCGCAAGAACCTGATGGACA (SEQ ID NO:7) |
| *Cre*-reverse | CTAGAGCCTGTTTTGCACGTTC (SEQ ID NO:8) |
| *Tomato*-forward | CTGTTCCTGTACGGCATGG (SEQ ID NO:9) |
| *Tomato*-reverse | GGCATTAAAGCAGCGTATCC (SEQ ID NO:10) |
| *SF_insert*-forword | ATGCCCTGGCTCACAAATAC (SEQ ID NO:11) |
| *SF_insert*-reverse | GCCAACCTTTGTTCATGGCA (SEQ ID NO:12) |
| *SF_donor_Tg*-forword | TTGCCGGGAAGCTAGAGTAA (SEQ ID NO:13) |
| *SF_donor_Tg*-reverse | TTTGCCTTCCTGTTTTTGCT (SEQ ID NO:14) |
| *SF_Rosa5'check_Tg*-forword | TCTTTTCTGTTGGACCCTTACCTTGACC (SEQ ID NO:15) |
| *SF_Rosa5'check_Tg-reverse* | ACGTCAATGGAAAGTCCCTATTGGCGTTAC (SEQ ID NO:16) |
| *SF_Rosa3'check_Tg*-forword | TGCCATGAACAAAGGTTGGCTATAAAGA (SEQ ID NO: 17) |
| *SF_Rosa3'check_Tg*-reverse | AGGATAGTGCAGGGAAACCCAAAGAAGT (SEQ ID NO:18) |

**Table 2**

| Gene Name | **Taqman probe** |
|---|---|
| *Col1a1* | Mm00801666_gl |
| *Col3a1* | Mm01254476_m1 |
| *Gata4* | Mm00484689_m1 |
| *Hand2* | Mm00439247_ml |
| *Mef2c* | Mm01340842_ml |
| *Nppb* | Mm01255770_g1 |
| *Tbx5* | Mm00803518_m1 |
| *Tgfb1* | Mm01178820_ml |

**Table 3**

| Gene Name | Primer Sequence | **Universal Probe Library** |
|---|---|---|
| *Gapdh-*forward | AGCTTGTCATCAACGGGAAG (SEQ ID NO:1 9) | #9 |
| *Gapdh-*reverse | TTTGATGTTAGTGGGGTCTCG (SEQ ID NO: 20) | |

### Isolation and Collection of Non-cardiomyocytes and Tomato-positive Cells

After euthanizing a mouse with CO₂ inhalation, the heart was immediately cannulated and perfused with cooling PBS (50 mL). After extracting the heart, the atrium and valve were separated to separate the ventricles, and the ventricular myocardium was cut into pieces of approximately 1 mm on a sterile Petri dish on ice. The pieces were transferred to a 10 mL tube containing an enzyme solution (3 mL) and incubated in a 37°C water bath for 45 minutes. The enzyme solution was pipetted every 15 minutes. The enzyme solution contained 2 mg/mL collagenase type IV (Worthington Biochemical, CLS-4) and 1.2 U/mL Dispase II (Sigma, 255-914-4). After final pipetting, the cell suspension was filtered with a 40 µm cell strainer. After the debris was removed by centrifugation using a debris removal solution (Miltenyi Biotec, 130-109-398), the red blood cells were removed using RBC lysis buffer (pluriSelect, 60-00050-11). The cell suspension was stained using Live/Dead (Invitrogen, L34975, 1:1000) Calcein Violet Working solution (BioLegend, 425203, 0.1 µm). Using a FACS device, cells without cell membrane damage and with metabolic activity retained (Live/Dead-, Calcein+) were collected while minimizing pressure on the cells. BD FACSAria^{™} IIIu (BD) and MoFlo XDP (Beckman Coulter) were used for FACS. When collecting Tomato-positive cells, the Tomato-positive cells were collected by FACS without cell staining.

### Single-cell RNA Sequencing

For single-cell RNA sequencing (scRNA-seq), analysis was performed on samples of each group with n = 2. The scRNA-seq library from non-cardiomyocytes was created using a Chromium Controller (10X Genomics). Approximately 10,000 cells were loaded onto each channel of a dedicated plate and processed using Chromium Single Cell 3' v3.1 reagent kit (10X Genomics). Sequencing was performed on a NovaSeq 6000 system (Illumina) operated by the Center for Omics and Bioinformatics, Graduate School of Frontier Sciences, The University of Tokyo.

The sequence reads were processed using a Cell Ranger v.5.0.0 pipeline (10X Genomics) to create a FASTQ file. In brief, a demultiplexed FASTQ file was aligned to a custom reference genome in which the tdTomato sequence was added to the mm10/GRCm38 reference genome, and a gene expression matrix was generated using a Cell Ranger count pipeline. After Cell Ranger count processing, the scRNA-seq data was analyzed using Seurat version 4.0.1 (Cell, 2021) in R version 4.0.3. Cells with gene expression of 200 or less or 5000 or more or with 10% or more of reads mapped to mitochondria were excluded to remove low-quality cells. After removal of low-quality cells, the expression values were normalized (NormalizeData function; scale.factor=10,000). The IntegratedData function was used to generate an integrated Seurat object with corrected differences between data sets. The dimensional reduction was performed using the uniform manifold approximation and projection (UMAP) algorithm with the RunPCA and RunUMAP functions implemented in the Seurat package. After dimensional reduction of the integrated Seurat object with dims = 1:30 and resolution = 0.25, the cell type of each cluster was identified based on the expression of known marker genes. Three clusters (an erythrocyte cluster, a platelet cluster, and a cluster with suspected dead cells) exhibiting a very low gene expression level were excluded from further analysis. The DimPlot function was used for cluster visualization after dimensional compression. CellChat (Nature Communications, 2021) Package was used to analyze cell-cell interaction. The FindMarkers function was used to compare gene expression differences between subclusters. In fibroblasts clusters, gene clusters that significantly increase more in an HFpEF group than in a normal chow group were selected based on criteria with a Bonferroni multiple adjusted p-value of less than 0.05 and an average logFC of 0.2 or more. The comprehensive average score of multiple gene clusters was calculated using the AddModuleScore function. The VlnPlot function was used for visualization. The DoHeatmap function was used to compare, between the samples, expression values of the representative gene clusters that are activated by cardiac impairment, and to visualize the expression values.

### ATAC Sequencing

ATAC sequencing was performed on each group with n = 3. Using the method described above, Tomato-positive cells were isolated and collected from the heart in vivo. To prepare the nucleus, 50,000 cells from Tomato positive cells were counted and centrifuged at 500 g for 5 minutes. Then, the cells were washed with cooling PBS and centrifuged again at 500 g for 5 minutes. The cells were dissolved in a solution containing 0.1% NP40, 0.1% Tween 20, and 0.01% digitonin. Immediately after dissolution, the nucleus was centrifuged at 500 g for 10 minutes using a refrigerated centrifuge. After discarding the supernatant, the nuclear pellets were resuspended in a transpose reaction mix (25 µL 2×TD buffer, 2.5 µL transpose, and 22.5 µL nuclease-free water). The transpose reaction was performed at 37°C for 30 minutes. Immediately after the transpose reaction, the sample was purified using a MinElute kit (QIAGEN, 28004). After purification, library fragments were amplified using 1x NEBnext PCR master mix and 1.25 µm custom Nextera PCR primers. To reduce GC and size bias in PCR, the PCR reaction was monitored using qPCR to stop amplification prior to saturation. For this qPCR, after five cycles of amplification of the whole library, 5 µl of the PCR reaction product was taken, and a 10 µl PCR cocktail containing SYBER Green at a final concentration of 0.6x was added. This reaction was performed for 20 cycles to determine the number of additional cycles required for the reaction of the remaining 45 µL of the PCR reaction product. The library was purified using a MinElute kit. Finally, the double-size selection was performed using SPRIselect (Beckman Coulter, B23317) to remove fragments of more than 1000 bp and fragments of less than 100 bp. Library sequencing was performed using HiSeq X Ten (Illumina). The adapter sequence was removed using fastp software (Chen et al., 2018). First, reads were aligned to mm10 genome using Bowtie2 according to the following parameters: --no-mixed - -no-discordant-X 2000. Duplicate reads were removed using Picard (https://broadinstitute.github.io/picard/). The BAM file was converted to a bigWig file using deepTools bamCoverage according to following parameters: -bs 1 -of bigWig --normalizeUsing CPM (Ramirez et al., 2016). Peaks were called using MACS2 (Zhang et al., 2008) with parameters set to "--nomodel --shift -50 -- extsize 100". Among the samples (n = 3), peak detected in the two replicates were defined as reliable peaks and used for further analysis. The coverage of each peak was calculated using featureCounts (Liao et al., 2014). A likelihood ratio test was performed using edgeR to detect peaks whose accessibility changes due to transcription factor processing or overexpression, and the significance was determined as p < 0.01. Motifs enriched in peaks with changed accessibility were detected using HOMER (Heinz et al., 2010). The genes nearest to the peaks were detected using HOMER, and the gene ontology of the listed genes was analyzed using Metascape (Zhou et al., 2019). The original Python script and deepTools were used for visualization. To infer the direct binding sites of Gata4 within the peaks with changed accessibility, overlap analysis was performed using published Gata4 ChIP-seq data from fibroblasts (Hashimoto et al., 2019, GSM3067561).

### Cell Culture and Retroviral Vector Infection

In order to construct a pMXs retroviral vector, the Cre coding region was amplified with PCR and subcloned into a pMXs vector for transfection into Plat-E cells using Fugene 6 (Promega, E2691), whereby retroviruses were generated. A newly generated pMX-Cre vector was transduced into fibroblasts. 24 hours after infection, the medium was replaced with DMEM/M199 (11150-059; Gibco) supplemented with 20% FBS, and the cells were cultured at 37°C in 5% CO₂.

### Statistical Analysis

Statistical significance was examined using the Student's t-test between two groups, or one-way analysis of variance (ANOVA) followed by Tukey's or Dunnett's post hoc test between three or more groups. Two-way ANOVA with Turkey's post hoc test was used to assess temporal changes in echocardiography and the like. Differences between groups were regarded as significant at p < 0.05. Statistical analysis was performed using GraphPad Prism software.

### Results

### Generation of Labelable Mouse with Freely Reprogrammable Cardiac Fibroblasts

Three types of mice, including a Tcf21 iCre mouse, a CAG-CAT-MGTH2A mouse, and an R26 tdTomato mouse, were crossed to generate a direct cardiac reprogramming mouse that is a triple transgenic mouse in which direct cardiac reprogramming can be controlled by cardiac fibroblasts in vivo. Fig. 1 shows a Tcf21 iCre mouse. Fig. 2 shows a CAG-CAT-MGTH2A mouse. Fig. 3 shows an R26 tdTomato mouse.

The Tcf21 iCre mouse is a mouse in which Cre protein expression is driven by transcription factor 21 (Tcf21), which is specifically expressed in cardiac fibroblasts. In Fig. 1, "MeRCreMer" is a sequence in which a mutated estrogen receptor (MER) is combined with Cre recombinase.

The CAG-CAT-MGTH2A mouse is a mouse that expresses reprogramming factors MGTH (four factors including Mef2c, Gata4, Tbx5, and Hand2) in response to expression of Cre. In Fig. 2, the factors MGTH are arranged in the order of Mef2c, Gata4, Tbx5, and Hand2. CAG is composed of the cytomegalovirus enhancer fused with the chicken β-actin promoter. CAT is chloramphenicol acetyltransferase.

The R26 tdTomato mouse is a mouse that expresses fluorescent protein Tomato in response to expression of Cre. In Fig. 3, Rosa26 (R26) is a gene region on mouse chromosome 6. The region allows genes to be easily inserted thereinto and can constantly express the inserted protein. tdTomato (tandem dimer Tomato) is a sequence in which two monomers of red fluorescent protein Tomato are connected in tandem.

Tamoxifen administration to the direct cardiac reprogramming mouse induces Cre expression in the entire cardiac fibroblasts, leading to expression of the reprogramming factors and Tomato. Thus, upon tamoxifen administration, some of the cardiac fibroblasts become red fibroblasts, and some are induced into red cardiomyocytes. Using this mouse, HFpEF therapy and elucidation of molecular biological mechanisms were aimed at.

### Confirmation of Expression of Fluorescent Protein and Reprogramming Factors in Cardiac Fibroblasts

The heart of the direct cardiac reprogramming mouse one week after tamoxifen administration was immunostained. Fig. 4 shows observation results from an immunostained cardiac section. The fibroblasts in the cardiac interstitium were labeled in red.

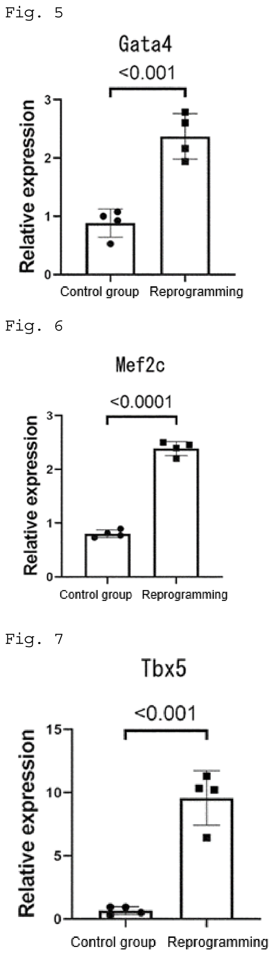

The heart of the direct cardiac reprogramming mouse one week after tamoxifen administration was also enzymatically treated to isolate cells. Then, fluorescent protein Tomato-positive cardiac fibroblasts isolated from the direct cardiac reprogramming mouse were collected using a fluorescence activated cell sorter (FACS). After RNA extraction, quantitative PCR was performed. Figs. 5 to 8 show the results. The control group shows the results from RNA samples of Tomato-positive cells collected from a Tcf21 iCre/R26 Tomato mouse that expresses only fluorescent protein Tomato and no reprogramming factors.

Figs. 5 to 8 confirmed that tamoxifen administration to the direct cardiac reprogramming mice induced Cre expression in the entire cardiac fibroblasts, leading to expression of the four reprogramming factors and Tomato.

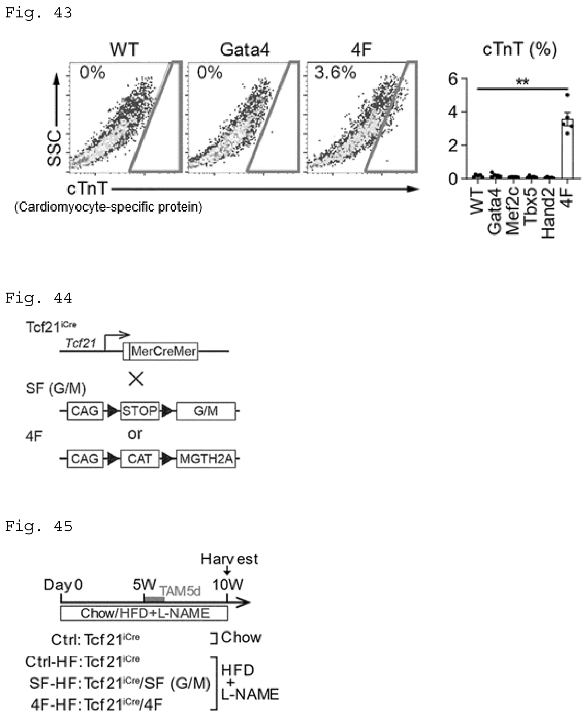

In Figs. 5 to 8, 18, 20, 22, 24, 26, and 29, the graph on the right side of Fig. 43, and Figs. 48, 49, 51, and 52, the points indicate individual values of the samples.

### Establishment of HFpEF Model and Examination of Therapeutic Effect

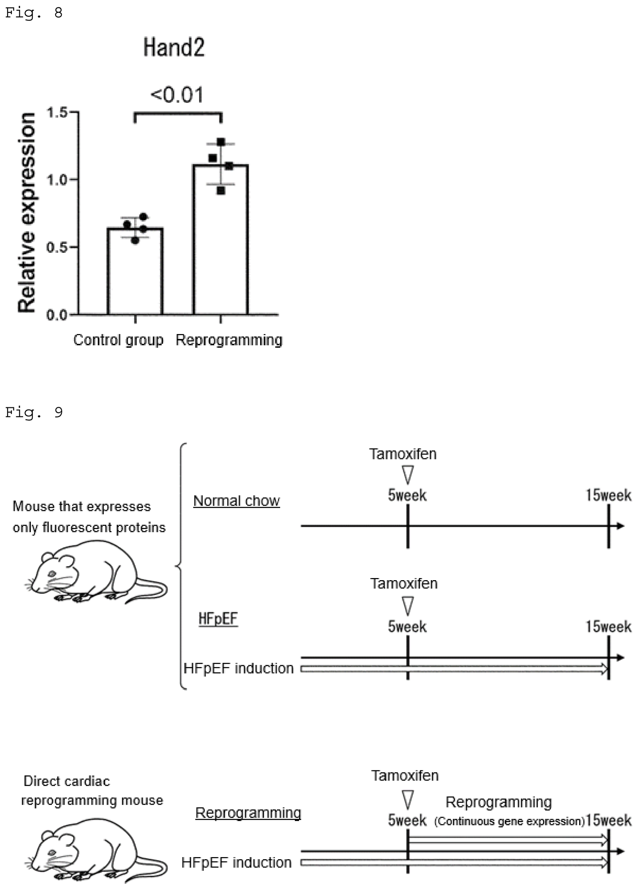

Using the direct cardiac reprogramming mice, HFpEF model was established to examine the therapeutic effect. HFpEF model was established with continuous ad libitum access to L-NAME water and a high-fat diet. The therapy group consisted of direct cardiac reprogramming mice. The control group consisted of R26 tdTomato mice, which only express fluorescent protein. The control group with normal chow was compared with the HFpEF-induced group, and the effect on the reprogramming therapy group for which reprogramming started after the onset of HFpEF was examined. Below, the normal chow control group is described as "normal chow," the HFpEF-induced control group is described as "HFpEF," and the HFpEF-induced direct cardiac reprogramming group is described as "reprogramming." Fig. 9 shows experimental protocols. The reprogramming genes were expressed in the reprogramming group by tamoxifen drug administration, with continuous HFpEF induction. The reprogramming genes were expressed continuously after tamoxifen administration.

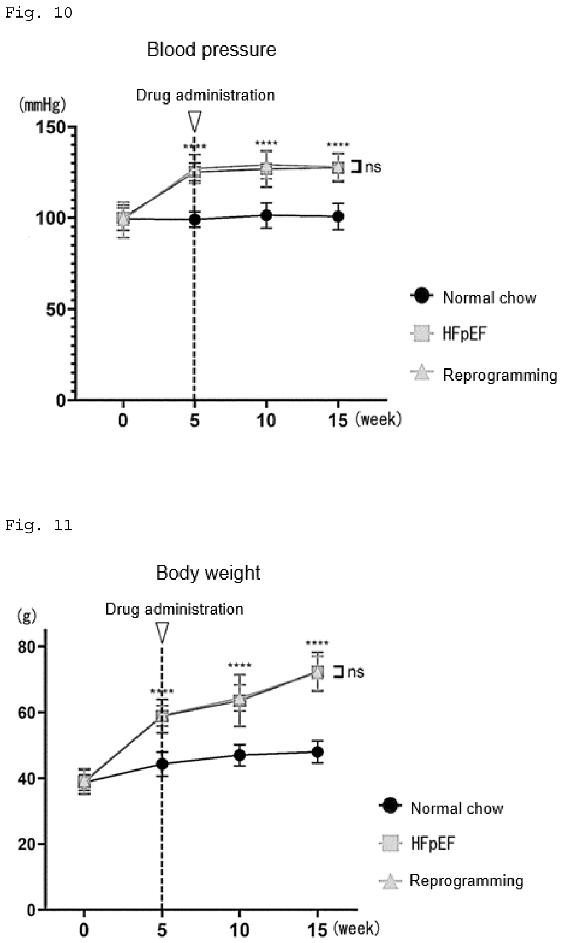

Fig. 10 shows systolic blood pressure measurement results from the normal chow group, the HFpEF group, and the reprogramming group. Fig. 11 shows body weight measurement results. "ns" indicates non-significant. "****" indicates p < 0.0001. In the HFpEF group and the reprogramming group, the systolic blood pressure and body weight were increased equally, and no significant difference was found between these groups.

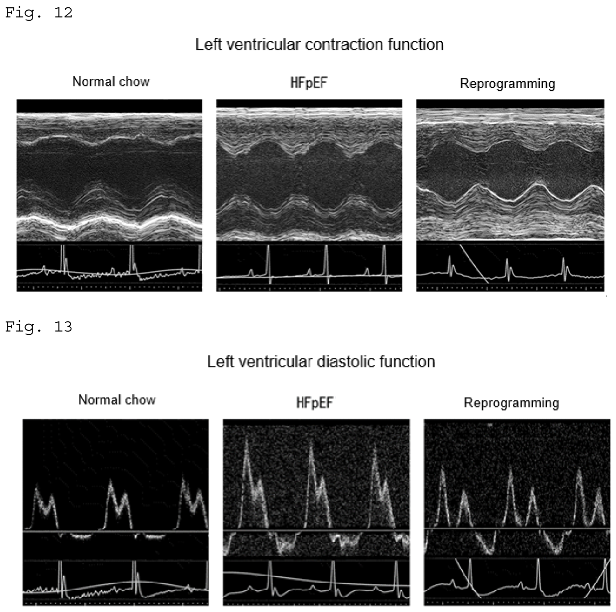

Fig. 12 shows temporal echocardiographic (M mode) observation results of left ventricular contraction. Fig. 13 shows temporal echocardiographic (Doppler mode) observation results of mitral valve inflow waveforms. The waveform usually includes two peaks. The first one corresponds to the E-wave (early diastolic phase) and the second one corresponds to the A-wave (atrial contraction). The ratio in height between the E-wave and the A-wave, E/A, is an indicator of left ventricular diastolic function. A higher ratio indicates poorer diastolic function. The quotient obtained by dividing the E-wave height by the tissue Doppler mitral annular early diastolic velocity E', E/E', also indicates the left ventricular diastolic function. A higher E/E' value also indicates poorer left ventricular diastolic function.

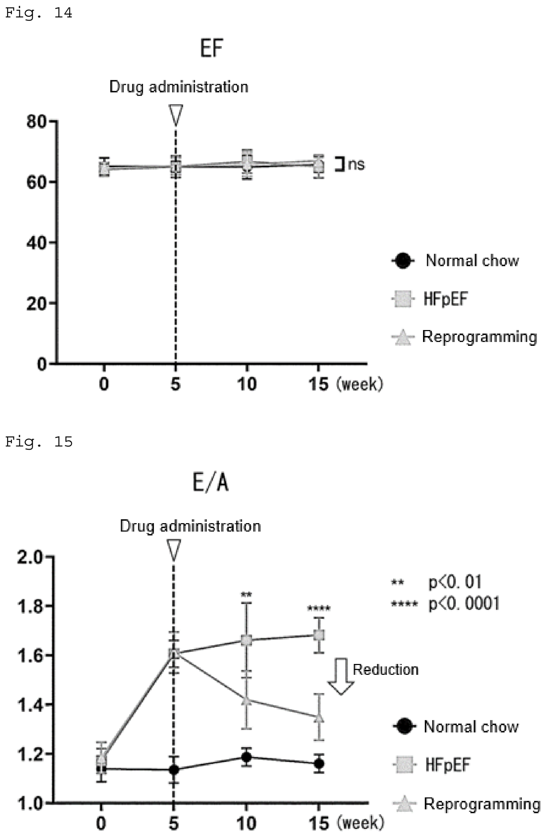

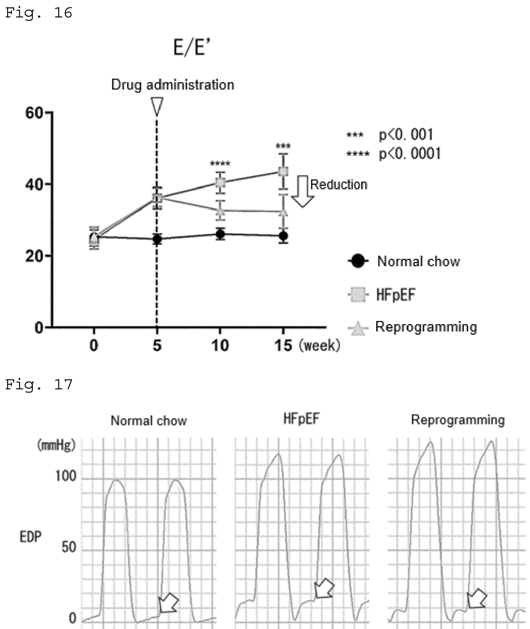

Figs. 14, 15, and 16 respectively show temporal changes in ejection fraction (EF), E/A, and E/E'. "ns" indicates non-significant. As shown in Fig. 14, none of the groups showed a reduction in EF from the baseline. In addition, as shown in Figs. 15 and 16, although HFpEF induction worsened the left ventricular diastolic function in the HFpEF group and the reprogramming group, the reprogramming group showed an improvement after the start of drug administration, compared to the HFpEF group.

### Diastolic Function Improvement by Reprogramming

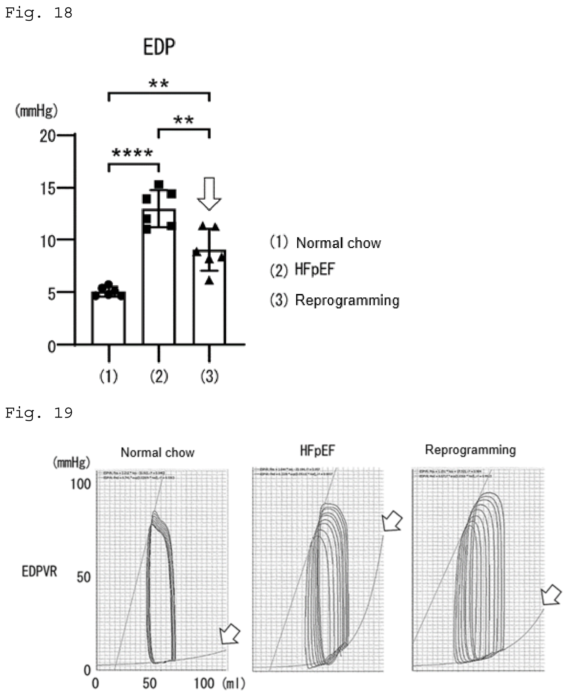

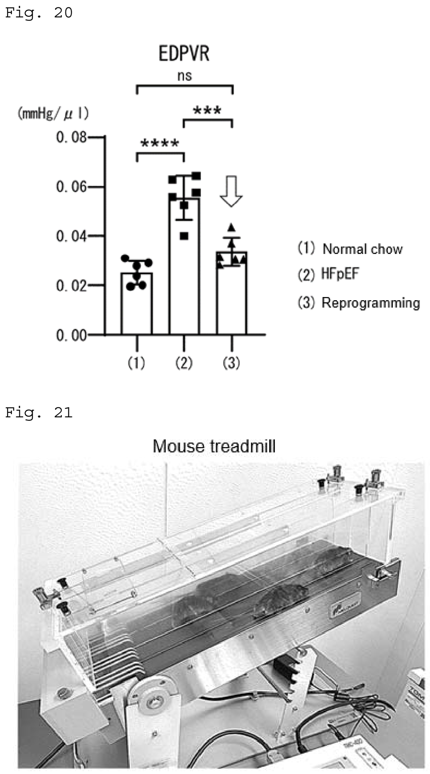

Figs. 17 to 20 show the results of mouse catheterization 15 weeks after the start of the experiment. Fig. 17 shows left ventricular pressure waveforms. The arrows indicate the left ventricular end-diastolic pressure (EDP). Fig. 18 is a bar graph showing EDP values. An increase in EDP is the gold standard indicator of heart failure. Fig. 19 shows PV loop (pressure-volume loop) waveforms. The arrows indicate end-diastolic pressure-volume relationship (EDPVR) curves. The slope of these curves reflect the left ventricular diastolic function. A steeper slope indicates a greater increase in pressure relative to the increase in capacity, indicating poor left vertical radial function. Fig. 20 is a bar graph showing EDPVR values. In Figs. 18 and 20, "ns" indicates non-significant, "**" indicates p < 0.01, "***" indicates p < 0.001, and "****" indicates p < 0.0001.

As shown in Figs. 18 and 20, while the HFpEF group showed a more significant increase in EDP and EDPVR than the normal chow group, the reprogramming group showed an improvement in EDP and EDPVR.

### Exercise Tolerance Improvement by Reprogramming

The exercise tolerance of the mice was evaluated at 15 weeks from the start of the experiment. Fig. 21 shows evaluations using a mouse treadmill. The mice ran on an inclined belt conveyor. The speed was gradually increased with time, and the exercise tolerance was evaluated based on the total running distance.

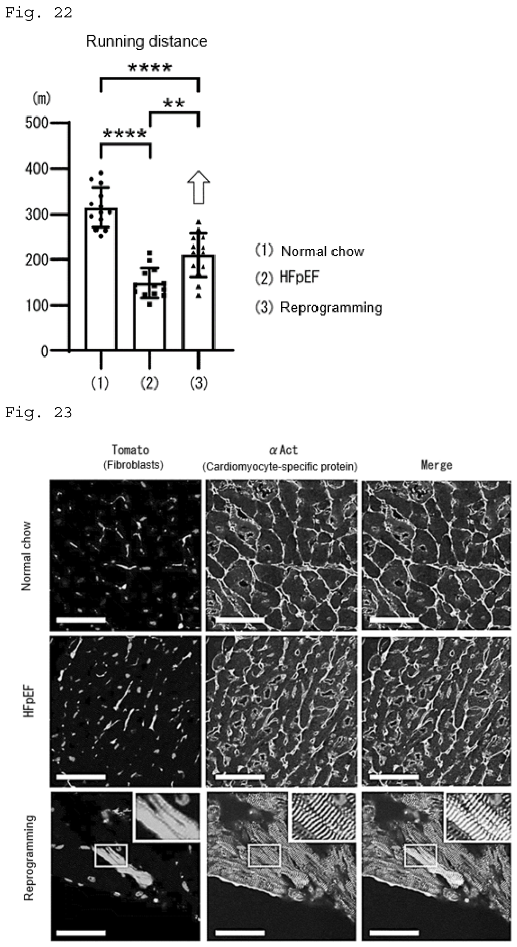

Fig. 22 shows the running distance of each group. In Fig. 22, "**" indicates p < 0.01, and "****" indicates p < 0.0001. As shown in Fig. 22, while the HFpEF group showed a reduction in running distance, the reprogramming group showed an improvement in running distance, which confirmed the effect of reprogramming therapy to improve the exercise tolerance. This indicates that the exercise tolerance was improved as a result of improved cardiac dilatation ability in the reprogramming group.

### Evaluation of Cardiomyocyte Induction Efficiency

The cardiomyocyte induction efficiency at 15 weeks from the start of the experiment was evaluated using immunostaining mouse cardiac sections. Fig. 23 shows immunostaining results. As shown in Fig. 23, in the reprogramming group, cardiomyocyte-specific protein expression was observed in some of the Tomato-positive cells, and regeneration into cardiomyocytes was confirmed. In addition, the reprogramming group shows clear horizontal patterns formed in cells with co-expressed Tomato and α-actin (α-Act).

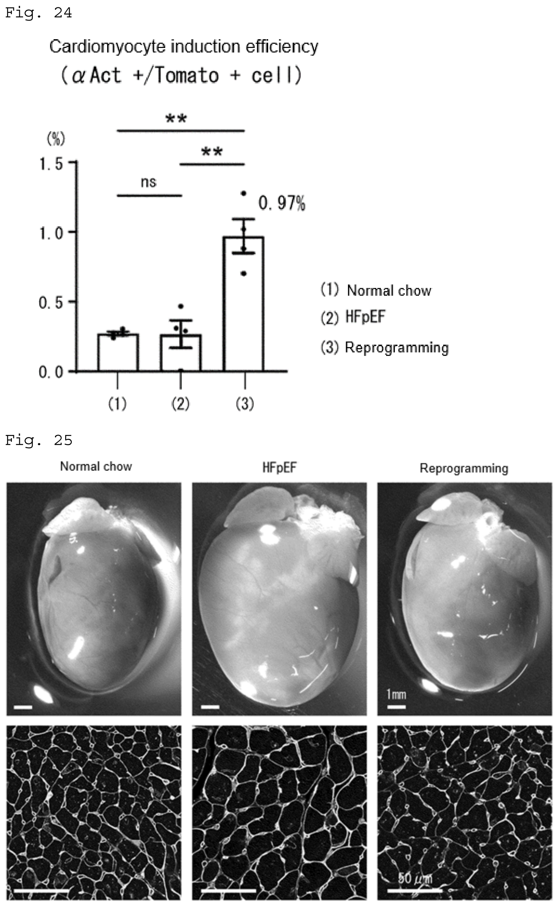

Fig. 24 shows the results obtained by quantifying the proportion of cells induced from fibroblasts to cardiomyocytes. "ns" indicates non-significant, and "**" indicates p < 0.01. As shown in Fig. 24, in the reprogramming group, approximately 1% fibroblasts were reprogrammed into cardiomyocytes.

### Amelioration of Cardiac Hypertrophy by Reprogramming

In Fig. 25, the upper row shows cardiac macroimages 15 weeks after the start of the experiment, and the lower row shows immunostained images of cardiac sections 15 weeks after the start of the experiment. Wheat germ agglutinin (WGA) was used to specifically stain the cell membrane green, whereby the borders of the cardiomyocytes became clearly visible, making it possible to measure the cardiomyocyte cross-sectional area. The cardiac macroimages show that while cardiac hypertrophy occurred in the HFpEF group, cardiac hypertrophy was ameliorated in the reprogramming group. The immunostained images also show an increase in cardiomyocyte size in the HFpEF group, and conversely, an improvement in cardiomyocyte size in the reprogramming group.

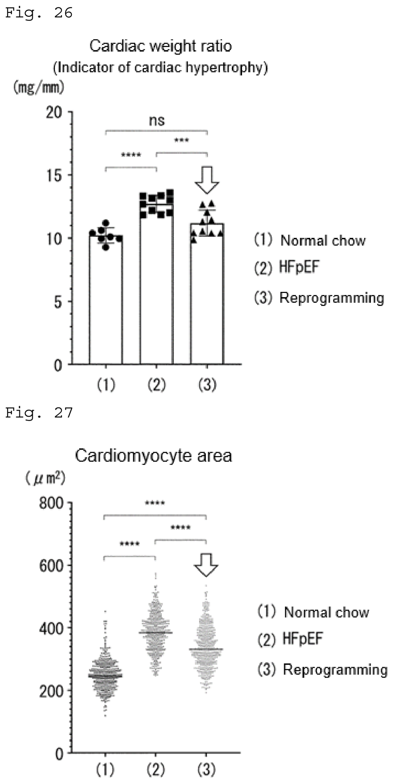

Fig. 26 shows measurement results of the ratio of cardiac weight corrected for femur length. "ns" indicates non-significant, "***" indicates p < 0.001, and "****" indicates p < 0.0001. The cardiac weight as an indicator of cardiac hypertrophy was corrected for femur length to reduce individual differences between mice. As shown in Fig. 26, while the cardiac weight was significantly increased in the HFpEF group, the increase in cardiac weight was reduced in the reprogramming group.

Fig. 27 shows cardiomyocyte area measurement results. "****" indicates p < 0.0001. The cross-sectional area of approximately 100 randomly selected cardiomyocytes per individual was measured. The results are shown as violin plots. While the HFpEF group showed an increase in cross-sectional area of the cardiomyocytes as a whole, the reprogramming group showed a reduction in area.

Cardiomyocyte hypertrophy is one of the causes of impaired cardiac diastolic function. The results in Figs. 25 to 27 show that inhibition of cardiomyocyte hypertrophy is associated with an improvement in cardiac diastolic function.

### Fibrosis Reduction by Reprogramming

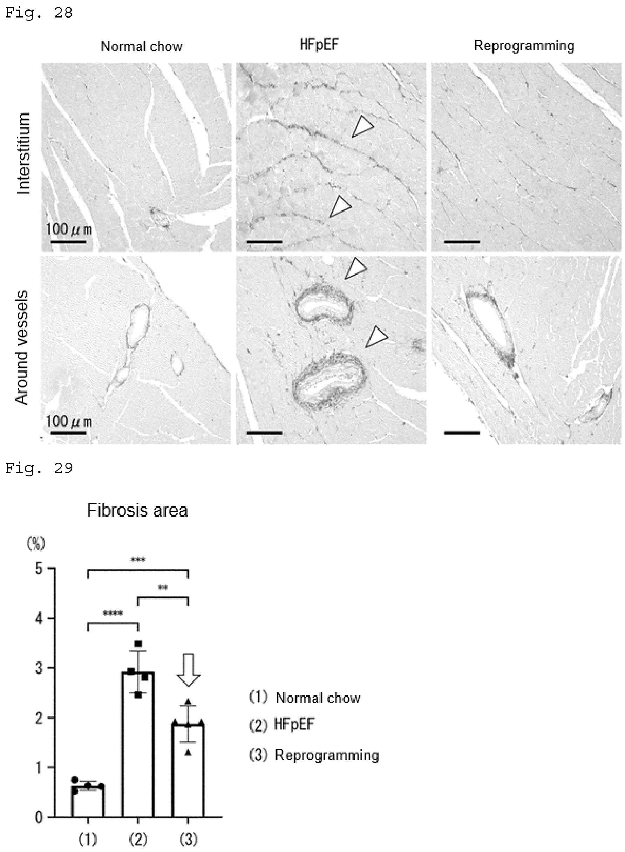

Fig. 28 shows cardiac section Sirius red staining results at 15 weeks from the start of the experiment. Sirius red staining stains fibrosis areas in red and tissues usually in yellow. For example, the arrowheads in Fig. 28 indicate the fibrosis areas. Clearly, fibrotic lesions in parts of the interstitium and around vessels were worsened in the HFpEF group. In contrast, the reprogramming groups showed a reduction in fibrosis area.

Fig. 29 shows the results of software analysis of the fibrosis area as the ratio of the fibrosis area stained in red to the total area of the tissue section observed. "**" indicates p < 0.01, "***" indicates p < 0.001, and "****" indicates p < 0.0001. The reprogramming group showed a significant reduction in fibrosis area compared to the HFpEF group.

Direct reprogramming of cardiac fibroblasts in the HFpEF mouse model was found to improve HFpEF due to ameliorated cardiac hypertrophy and reduced fibrosis. A breakdown of direct reprogramming showed that approximately 1% of fibroblasts activated by the disease were induced into cardiomyocytes. At the same time, the changes throughout the heart were diffuse, and a hypothesis was established that the therapeutic effects of reprogramming were due not only to the cardiomyocyte induction in 1% of fibroblasts, but also due to the suppression of the remaining 99% of fibroblasts that were not induced into cardiomyocytes. Further analysis was conducted on the possible anti-fibrotic effect of direct cardiac reprogramming.

### scRNA-seq

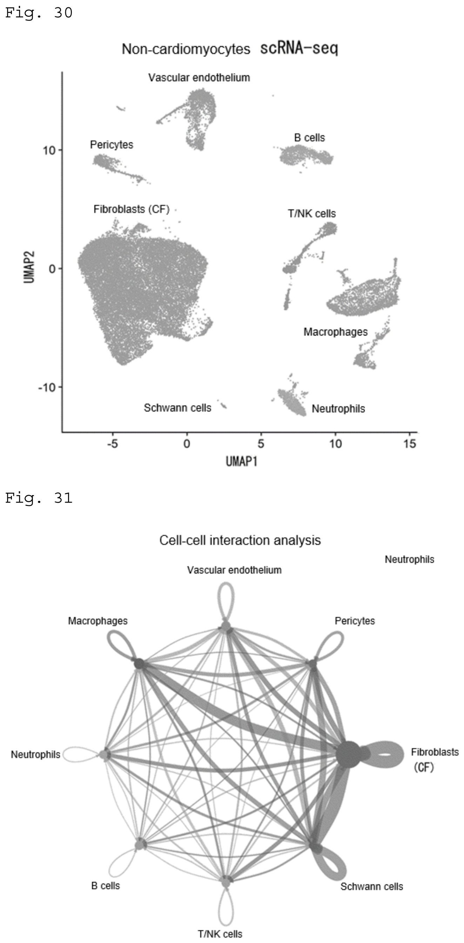

Fig. 30 shows single-cell RNA sequencing (scRNA-seq) results from non-cardiomyocytes in the heart. Each single dot indicates the information of one cell. Cells are classified into several clusters based on different patterns of gene expression. Cell species are identified based on a representative gene expression profile within each cluster. Fig. 30 shows that noncardiomyocytes in the heart were collected uniformly, including fibroblasts.

Fig. 31 shows ligand-receptor interaction analysis between the cell clusters shown in Fig. 30. The lines in Fig. 31 indicate cell-cell interaction. Thicker lines indicate stronger interactions. Fig. 31 shows that cardiac fibroblasts have a significant effect on other cells and that the fibroblasts have strong interactions with each other. Thus, fibroblasts that underwent gene expression therapeutic intervention were analyzed in detail.

### Improvement in Pathological Gene Expression Changes in Fibroblasts by Reprogramming

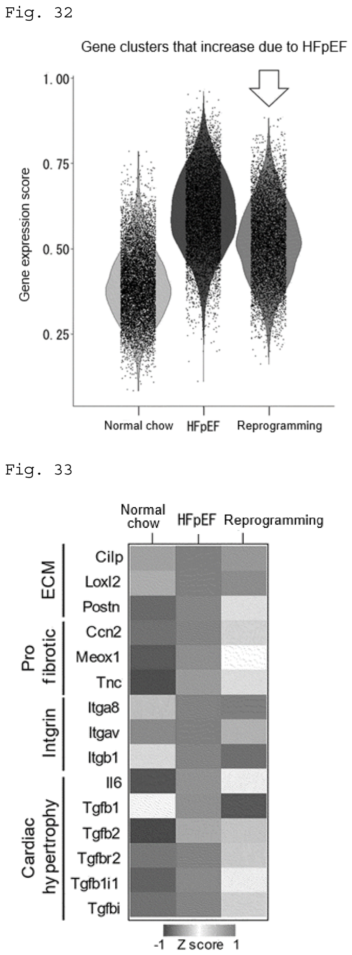

Fig. 32 shows the results obtained by extracting and analyzing only gene expression changes in the fibroblast cluster alone. In Fig. 32, the central violin plot shows a comprehensive score of pathogenic genes whose expression is increased due to HFpEF on the vertical axis. The reprogramming group showed an improvement in pathological gene expression changes that had been increased due to HFpEF.

Fig. 33 shows a heatmap for expression of representative gene clusters that are activated in response to cardiac impairment. The reprogramming group showed an improvement in, for example, expression of many fibrosis-related gene clusters, such as Il6 and Tgfb, associated with cardiac hypertrophy signaling.

### ATAC-seq

To analyze the effect of the expressed reprogramming genes on the therapeutic effect, an assay for transpose-accessible chromatin with high-throughput sequencing (ATAC-seq) was performed. In this assay, open chromatin regions were selectively fragmented using Tn5 transpose to construct a sequence library with tags added simultaneously. This technique enables evaluation of opening or closing of chromatin and inference of regulatory mechanisms upstream of gene expression.

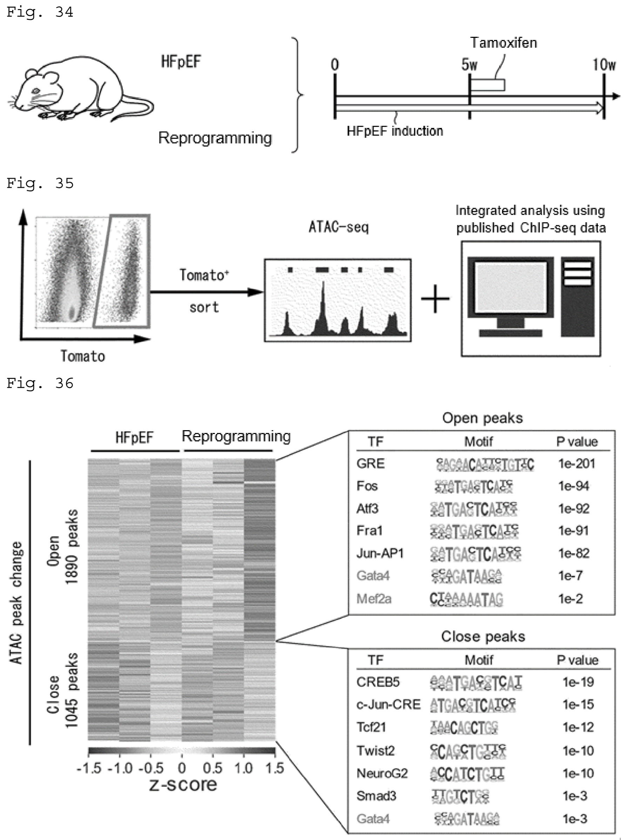

Figs. 34 and 35 show an ATAC-seq experimental workflow. As shown in Fig. 34, to evaluate the therapeutic mechanism, ATAC-seq was performed on two groups including the HFpEF group and the reprogramming group. Tomato-positive fibroblasts were collected from in vivo mouse heart and assayed at 5 weeks from tamoxifen drug administration (10 weeks from the start of the experiment). In addition, as shown in Fig. 35, integrated analysis using published ChIP-seq data was also performed to further explore direct binding of the reprogramming factors. Chromatin immunoprecipitation sequencing (ChIP-seq) is a method in which DNA complexes with antibodies are immunoprecipitated and the precipitated DNA fragments are sequenced to analyze which regions of DNA interacted with transcription factors.

Fig. 36 shows a heatmap for signal intensity in the region where the peak wave height was significantly different (ATAC peak change) in ATAC-seq. In both the HFpEF group and the reprogramming group, n = 3. Among the ATAC peak changes, the reprogramming group included 1890 peaks in open regions where peaks are high and 1045 peaks in close regions where peaks are low. GATA4-binding motifs were found in both the open and close regions as a result of motif analysis to assess how strongly known transcription factor binding gene sequences are enriched in each region. Mef2c, Tbx5, and Hand2 motifs were not found. This suggests that Gata4 may be an important factor involved in the effect on therapeutic effects in the reprogramming factor.

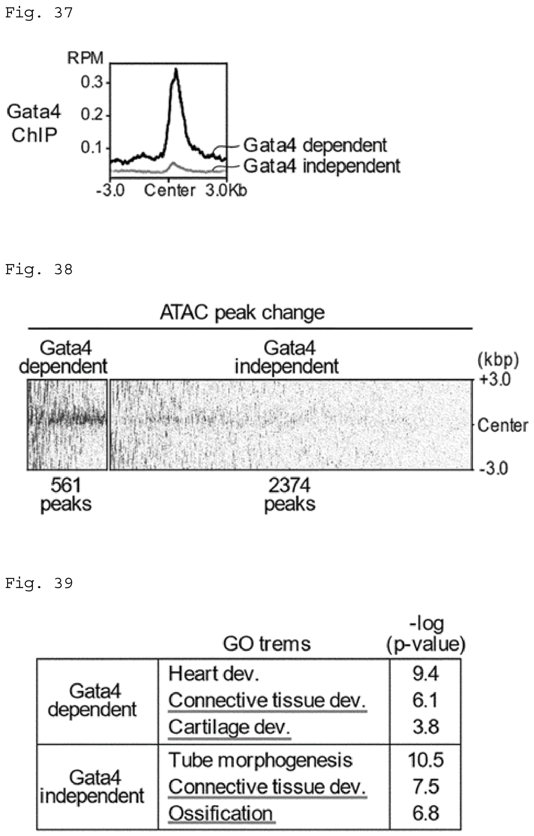

Among the analyzed ATAC-peak changes, the regions were classified into two regions: one with significant peaks (Gata4 dependent) that are also present in published fibroblasts Gata4 ChIP-seq data; and one without such significant peaks (Gata4 independent). Then, these regions were analyzed. Fig. 37 summarizes all of the peak heights that are categorized into Gata4 dependent and Gata4 independent.

Fig. 38 shows a heatmap for intensity of each peak signal. Among the ATAC peak changes, The Gata4 dependent region includes 561 peaks, and the Gata4 independent region includes 2374 peaks.

Gene ontology (GO) analysis was performed on genes near the respective peaks. Gene analysis (GO analysis) is analysis that annotates gene functions by focusing on known biological processes and molecular functions of input gene clusters. Fig. 39 shows the analysis results. As shown in Fig. 39, each was found to be a gene cluster involved in fibrosis. This suggests that Gata4 is directly and indirectly involved in therapeutic effect.

### Generation of Genetically Modified Mouse that Expresses Only Single Reprogramming Factor

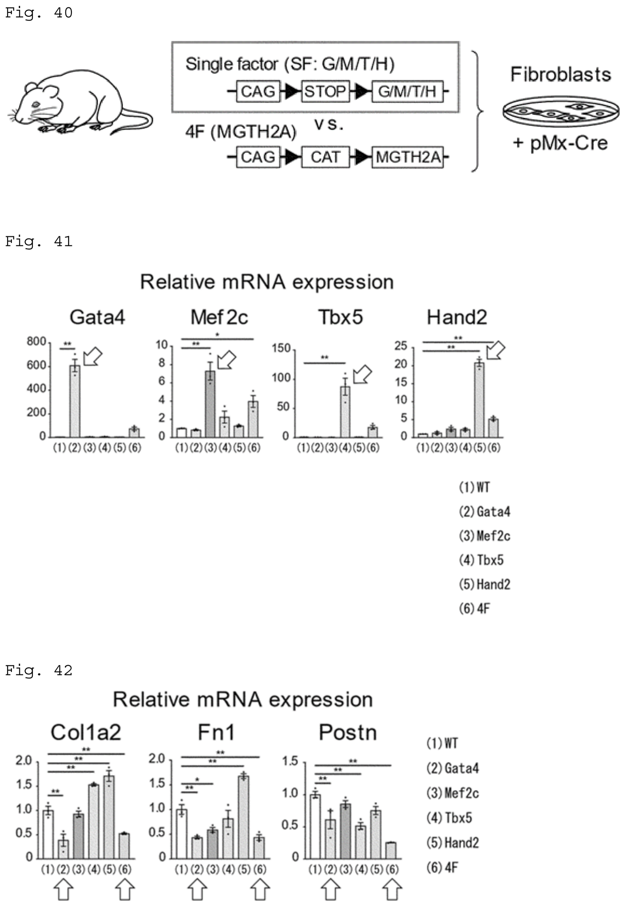

Based on the previous experiment results, the possibility or impossibility of HFpEF therapy with a single reprogramming factor was examined. Fig. 40 shows an overview of a genetically modified mouse. Four lines of single factor mice each in which the STOP sequence flanked by loxp sequences and one of Gata4, Mef2c, Tbx5, and Hand2 factors were arranged at downstream of the CAG promoter were generated. In addition to these four types of single factor mice, the direct cardiac reprogramming mouse used so far was named 4F mouse and analyzed simultaneously.

Fetal fibroblasts were collected from these genetically modified mice, and each target factor was expressed in cell dishes by forced Cre expression using retrovirus pMx-Cre. Fig. 41 shows a graph of quantitative PCR on RNA from fetal fibroblasts (wild type, each of four single factor mouse strains, and 4F mouse fibroblasts). It shows that while all of the factors, i.e., Gata4, Mef2c, Tbx5, and Hand2, were forcibly expressed in 4F, only one factor was forcibly expressed in the corresponding single factor group. Importantly, as indicated by the arrows, the expression of only one factor was enhanced, without increasing the expression of other endogenous factors.

Fig. 42 is a quantitative PCR graph of RNA from fetal fibroblasts one week after infection with pMx-Cre. Quantitative PCR was performed on well-known fibrosis genes, Col1a2, Fn1, and Postn. As indicated by the arrows, only Gata4 single factor and 4F showed a reduction in all of these fibrosis genes compared to wild-type fibroblasts.

Fig. 43 shows FACS results one week after infection of fetal fibroblasts with pMx-Cre. Cardiomyocyte-specific protein troponin T (cTnT) was immunostained to assess the positive rate. cTnT became positive only in 4F. Expression of a single factor alone, such as Gata4 single factor, did not result in induction into cardiomyocytes. In the graph, "**" denotes p < 0.01.

It has been reported that when performing direct cardiac reprogramming using four or more reprogramming factors, reprogramming by a polycystronic vector carrying multiple reprogramming factors as a single gene can achieve higher-quality cardiomyocyte regeneration, as opposed to reprogramming by a vector carrying each reprogramming factor individually (Kohei Inagawa et al., Circ Res. 2012 Oct 12; 111(9): 1147-56). According to this report, efficient therapy requires uniform introduction of four or more genes.

Meanwhile, another report has been made on gene therapy, regarding the size of the gene that can be carried by a clinically applicable and safe vector for use in humans (Kenneth Lundstrom, Diseases 2018, 6, 42; Clare E. Thomas et al., Nat Rev Genet. 2003 May, 4(5): 346-58; Takehiro Ura et al., Vaccines 2014, 2, 624-641). The above four or more reprogramming factors are larger than the size of the gene that can be carried by a clinically applicable and safe vector for use in humans, and the technology of a safe polycystronic vector that can uniformly introduce four or more genes has not been developed.

Based on such background art, it is desirable to develop a therapy using a single gene with a size that can be carried by a safe vector. Thus, the HFpEF therapeutic effect of a single reprogramming factor was examined.

### Examination of HFpEF Therapeutic Effect of Gata4 Single Factor

To examine the HFpEF therapeutic effect of Gata4 single factor, an in vivo mouse experiment was performed. Fig. 44 shows an overview of genetically modified mice used. A Tcf21^{iCre} mouse in which Cre protein expression is driven by Tcf21, which is specifically expressed in cardiac fibroblasts, was crossed with a single factor mouse in which a single reprogramming factor is expressed in response to Cre expression or a 4F mouse in which four reprogramming factors (MGTH) are expressed in response to Cre expression, and the thus-obtained mouse was used. In addition to a Gata4 single factor mouse, a Mef2c single factor mouse, which has been reported to be an important reprogramming factor in cardiomyocyte induction, was also used to confirm that the effect is not solely attributable to the mere expression of the factor. In the generated mouse, a single factor is expressed specifically in cardiac fibroblasts.

Fig. 45 shows experimental protocols. Only mice with gene expression of Tcf21^{iCre} only were used in the following two groups: the control group (Ctrl) with normal chow; and the HFpEF-induced group (Ctrl-HF). HFpEF induction was sustained in both Tcf21^{iCre}/single factor (Gata4 or Mef2c) mice (SF-HF (Gata4-HF or Mef2c-HF)) and Tcf21^{iCre}/4F mice (4F-HF). The factor-based therapy was performed with tamoxifen drug administration at 5 weeks from the start of the experiment, and analysis was performed at a total of 10 weeks.

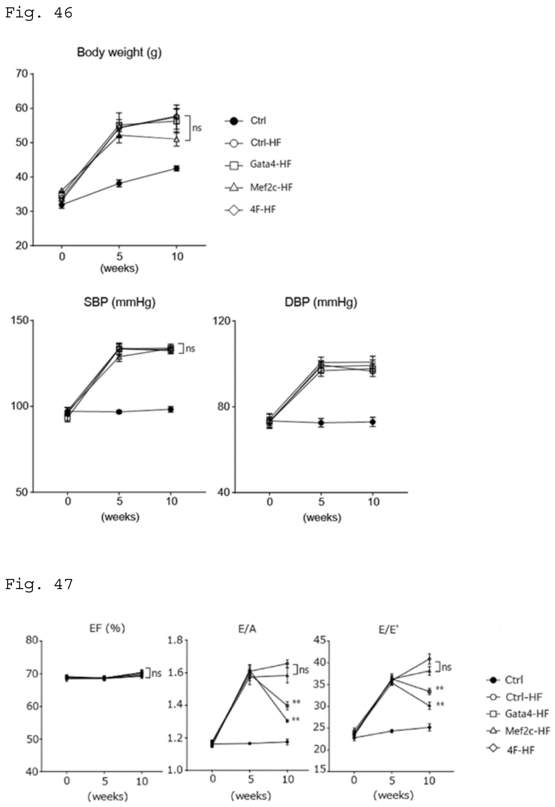

Fig. 46 shows temporal changes in body weight, systolic blood pressure (SBP), and diastolic blood pressure (DBP) as indicators of HFpEF induction. The horizontal axis shows weeks. "ns" indicates non-significant. No difference was found in body weight, SBP, and DBP in the groups with HFpEF induction.

Fig. 47 shows echocardiographic results. "ns" indicates non-significant, and "**" indicates p < 0.01. All of the groups showed no change in ejection fraction (EF) as an indicator of cardiac contractility. While E/A and E/E' as the indicators of left ventricular diastolic function were worsened in all of the groups subjected to HFpEF induction, Gata4-HF and 4F-HF showed an improvement after the start of drug administration. Importantly, there was no significant improvement in the case of Mef2c alone.

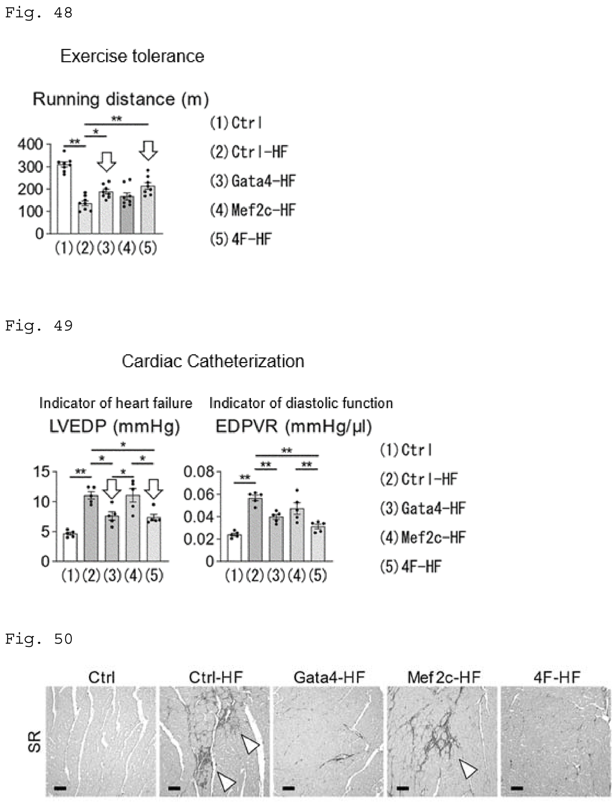

Fig. 48 shows the treadmill running distance (m) as an indicator of exercise tolerance at 10 weeks from the start of the experiment. "*" indicates p < 0.05, and "**" indicates p < 0.01. Although HFpEF induction reduced exercise tolerance, Gata4-HF and 4F-HF showed a significant improvement.

Fig. 49 shows cardiac catheterization results at 10 weeks from the start of the experiment. "*" indicates p < 0.05, and "**" indicates p < 0.01. The left ventricular end-diastolic pressure (LVEDP) as the indicator of heart failure and the end-diastolic pressure-volume relationship (EDPVR) as the indicator of left ventricular diastolic function were worsened by HFpEF induction. Only two groups, Gata4-HF and 4F-HF, showed a significant improvement.

Fig. 50 shows cardiac section Sirius red staining results at 10 weeks from the start of the experiment. While the fibrosis area was increased in Ctrl-HF and Mef2c-HF compared to Ctrl, the fibrosis area was reduced in Gata4-HF and 4F-HF.

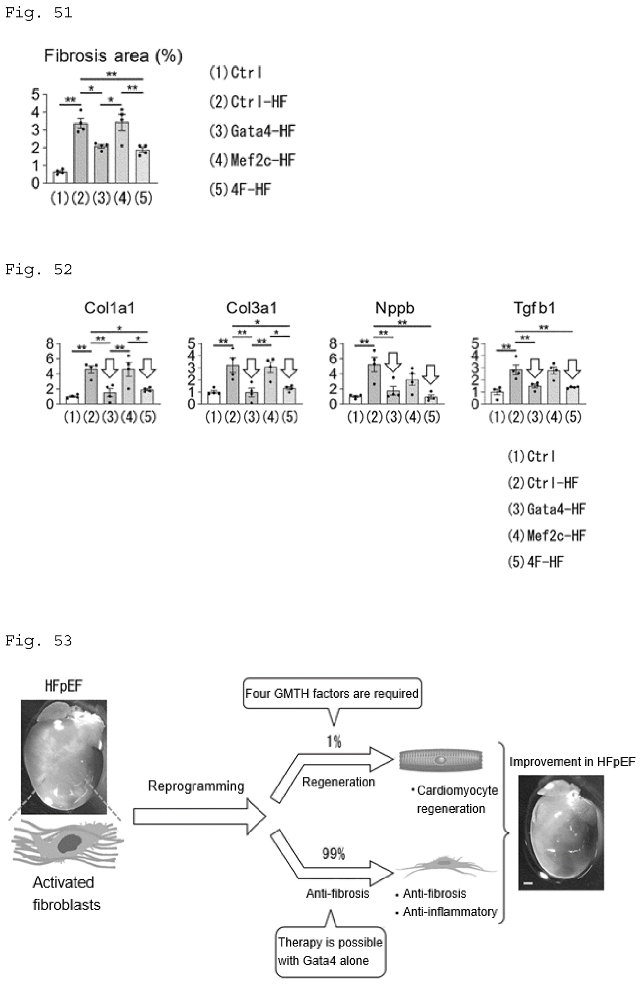

Fig. 51 shows a graph of fibrosis area analyzed. "*" indicates p < 0.05, and "**" indicates p < 0.01. Gata4-HF and 4F-HF showed a significant improvement in fibrosis area that had been increased due to HFpEF.

Fig. 52 shows quantitative PCR results from RNA extracted from both ventricles of the mouse heart removed at 10 weeks from the start of the experiment. Gene expression of Col1a1 and Col3a1 indicating cardiac fibrosis, Nppb as an indicator of heart failure, and Tgfb1 representing fibrosis and hypertrophy signaling were increased due to HFpEF induction. While Gata4-HF and 4F-HF showed an improvement in expression of these genes, Mef2c-HF showed no improvement.

### Conclusions

Fig. 53 summarizes the results of the Example. While fibroblasts are activated in HFpEF, direct reprogramming was found to cause cardiomyocyte regeneration from 1% fibroblasts. Considering the latest results of gene expression analysis, it was found that the remaining 99% fibroblasts that do not undergo cardiomyocyte regeneration exert anti-fibrotic effect, comprehensively improving HFpEF. Analysis on a single factor showed that while the four GMTH factors are required for cardiomyocyte regeneration as known, the therapeutic effect for anti-fibrosis can also be achieved with Gata4 alone, improving cardiac diastolic function in HFpEF. This Example shows that gene therapy using Gata4 alone can be a means for HFpEF therapy.

### Industrial Applicability

The present invention can be used, for example, in therapy, such as gene therapy of heart failure (in particular, HFpEF) .

## Claims

1. A cardiac diastolic function-improving agent comprising a polynucleotide encoding a reprogramming factor polypeptide Gata4.

2. The cardiac diastolic function-improving agent according to claim 1, wherein the reprogramming factor polypeptide Gata4 comprises an amino acid sequence having at least 90% identity to the amino acid sequence represented by SEQ ID NO: 1 or SEQ ID NO: 3.

3. The cardiac diastolic function-improving agent according to claim 1, wherein the polynucleotide comprises a nucleotide sequence having at least 90% identity to the nucleotide sequence represented by SEQ ID NO: 2 or SEQ ID NO: 4.

4. The cardiac diastolic function-improving agent according to any one of claims 1 to 3, which is an agent for improving heart failure.

5. The cardiac diastolic function-improving agent according to claim 4, wherein the heart failure is heart failure with preserved ejection fraction (HFpEF).

6. A cardiac diastolic function-improving agent comprising a factor that upregulates the expression of Gata4 gene.

7. The cardiac diastolic function-improving agent comprising a factor that increases the expression level of Gata4 gene in cardiac fibroblasts.

8. A screening method for a cardiac diastolic function-improving agent, comprising:
contacting a test substance with fibroblasts; and
evaluating the expression of Gata4 gene in the fibroblasts.
